# EUROPEAN PATENT SPECIFICATION

(11) **EP 3 984 514 B1**
(45) Date of publication and mention of the grant of the patent: **10.07.2024**
(21) Application number: 20212757.7
(22) Date of filing: 09.12.2020
(51) Int. Cl.: A61H 7/00, A61H 23/02, A61H 15/00, A61N 1/36

(54) **ACOUSTIC WAVE VIBRATION APPARATUS AND APPLICATION INCLUDING THE SAME**
SCHALLWELLENSCHWINGUNGSVORRICHTUNG UND ANWENDUNG DAVON
APPAREIL DE VIBRATION D'ONDES ACOUSTIQUES ET APPLICATION LE COMPRENANT

(30) Priority: 16.10.2020 KR 20200134245
(43) Date of publication of application: 20.04.2022
(73) Proprietor: Evosonics Co., Ltd., Gangwon-do 26311 (KR)
(72) Inventor: CHOE, Jae Yeung, Gangwon-do 26316 (KR)
(74) Representative: Brann AB

(56) References cited:
- WO-A1-2016/085301
- KR-A- 20140 003 990
- KR-A- 20150 087 897
- KR-A- 20160 140 176
- US-A1- 2015 297 393

## Description

### BACKGROUND

### 1. Field of the Invention

The present invention relates to a vibration module for transmitting acoustic wave vibration to a human body, a vibration apparatus, and an application in which the vibration module is mounted, and more specifically, to a vibration module that can vibrate using acoustic pressure for efficient vibration transmission, a vibration apparatus, and an application in which the vibration module is mounted.

### 2. Discussion of Related Art

Recently, there is a boom in home training for exercising at home. As exercise apparatuses for such home training, there are simple apparatuses such as foam rollers, balance cushions, etc. and demand for such exercise apparatuses is recently increasing. In addition, as a method of preventing and treating hemorrhoids, prostatitis, or gynecological diseases at home or in a clinic, rehabilitation treatment apparatuses that help blood circulation by keeping a perineal region warm or provide vibration around the perineal region and massage are used.

Meanwhile, the conventional foam rollers, balance cushions, or rehabilitation treatment apparatuses do not provide vibration, use motor vibration even when vibration is provided, or use a method in which a vibration module directly hits the human body.

Such motor vibration has a risk of damaging muscles or nerves due to burdensome stimulation to the human body, and there is a limitation in that the vibration cannot be transmitted uniformly to the entire human body.

In the present invention, a vibration module, a vibration apparatus, and an application including the same are provided to solve such conventional problems.

KR 2015 0087897 discloses a local vibrator using sound waves. According to an embodiment, the local vibrator comprises: a body which has a storage space therein, composed of a lower body and an upper body, and has band members on both sides of the upper body; a fixating plate installed between the lower body and the upper body, where an upper central unit is positioned inside the upper body; a supporting member positioned inside the storage space of the lower body whose upper part is bonded to the fixating plate, and where a permanent magnet is fixated to the inner bottom; a ring-shaped cone damper guide bonded to the upper part of the supporting member; a cone damper screwed to the cone damper guide in order to play an original sound, is shaped in a disc, and has a plurality of dampers along the edge including a voice coil on the lower part thereof; a sound wave transmitting probe coupled to the lower part of the cone damper, whose upper part is exposed to the outside of the upper body in order to transmit vibration generated from the cone damper to a human body; and a battery positioned on the lower part of the supporting member.

### SUMMARY OF THE INVENTION

The present invention is directed to a vibration apparatus according to claim 1. Subsidiary aspects of the invention are provided in the dependent claims.

### SUMMARY OF THE DISCLOSURE

The present invention is directed to providing a vibration module that can vibrate using acoustic pressure.

The present invention is also directed to providing an elastic member including a damper including a plurality of bending points, and a vibration module including the same.

The present invention is also directed to providing an elastic member including a damper having a different radius of curvature for each part, and a vibration module including the same.

The present invention is also directed to providing a vibration module including a plurality of elastic members.

The present invention is also directed to providing various applications that can vibrate using acoustic pressure.

The present invention is also directed to providing an acoustic wave vibration apparatus that performs a vibration frequency adjustment function and a cooling and heating function.

Objects of the present invention are not limited to the above-described objects and other unmentioned objects may be clearly understood by those skilled in the art from this specification and the accompanying descriptions.

According to an aspect of the present disclosure, there is provided a vibration apparatus for generating vibration with acoustic wave, the vibration apparatus may comprise a vibration module comprising a vibration generating unit and a vibration amplification unit, and wherein the vibration generating unit includes a housing providing an inner space, a magnet located in the housing, a bobbin located apart from the magnet in a direction of axis of the magnet, a coil disposed at an outer peripheral surface of the bobbin and an elastic member located on one side of the bobbin, and wherein the vibration amplification unit includes a probe connected to the bobbin and vibrating in the vertical direction, a vibration transmission module configured to couple to the housing directly or indirectly and surround at least a part of the probe such that the vibration transmission module induces dispersion of external force to the probe, wherein the probe has a predetermined weight to amplify the vibration generated by the vibration generating unit.

According to an aspect of the present disclosure, there is provided a massage device with acoustic wave, the massage device may comprise a vibration module comprising a vibration generating unit and a vibration amplification unit, wherein the vibration generating unit includes a housing providing an inner space, a magnet located in the housing, a bobbin located apart from the magnet in a direction of axis of the magnet, a coil disposed at an outer peripheral surface of the bobbin and an elastic member located on one side of the bobbin, and wherein the vibration amplification unit includes a probe connected to the bobbin and vibrating in the vertical direction, a vibration transmission module configured to couple to the housing directly or indirectly and surround at least a part of the probe such that the vibration transmission module induces dispersion of external force to the probe, and a vibration receiving unit configured to surround at least part of the vibration transmission module, wherein the probe has a predetermined weight to amplify the vibration generated by the vibration generating unit, wherein, the vibration transmission module is contacted to the vibration receiving unit such that the vibration amplified by the vibration amplification unit transmits to the vibration receiving unit, and wherein, the vibration receiving unit is not directly connected to the vibration amplification unit.

According to an aspect of the present disclosure, there is provided an exercise apparatus using acoustic wave, the exercise apparatus may comprise a massage device with acoustic wave, the massage device comprising: a vibration module comprising a vibration generating unit and a vibration amplification unit, wherein the vibration generating unit includes a housing providing an inner space, a magnet located in the housing, a bobbin located apart from the magnet in a direction of axis of the magnet, a coil disposed at an outer peripheral surface of the bobbin and an elastic member located on one side of the bobbin, and wherein the vibration amplification unit includes a probe connected to the bobbin and vibrating in the vertical direction, a vibration transmission module configured to couple to the housing directly or indirectly and surround at least a part of the probe such that the vibration transmission module induces dispersion of external force to the probe, and a vibration receiving unit configured to surround at least part of the vibration transmission module, wherein the probe has a predetermined weight to amplify the vibration generated by the vibration generating unit, wherein, the vibration transmission module is contacted to the vibration receiving unit such that the vibration amplified by the vibration amplification unit transmits to the vibration receiving unit, wherein, the vibration receiving unit is not directly connected to the vibration amplification unit, and wherein, the vibration transmission module is located inside the vibration receiving unit.

Solutions of the present invention are not limited to the above-described solutions and other unmentioned solutions may be clearly understood by those skilled in the art from this specification and the accompanying descriptions.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a block diagram for describing components constituting an acoustic wave vibration apparatus according to a first embodiment.
FIG. 2 is a diagram of a configuration of a special effect chair according to an embodiment.
FIG. 3 is a diagram for describing an example of a shape of a head part of a probe according to an embodiment.
FIG. 4 is a block diagram for describing components constituting an acoustic wave vibration apparatus according to a second embodiment.
FIG. 5 is a diagram for describing components constituting a vibration module according to the second embodiment.
FIG. 6 is a diagram for describing a housing of a vibration module according to another embodiment.
FIG. 7 is a block diagram for describing components constituting an acoustic wave vibration apparatus according to a third embodiment.
FIG. 8 is a diagram for describing components constituting a vibration module according to the third embodiment.
FIG. 9 is a flowchart for describing a series of operations performed by a controller according to an embodiment.
FIG. 10 is a diagram illustrating an elastic member according to an embodiment.
FIG. 11 is a diagram illustrating an elastic member including a damper having parts that are bent in different directions for each part according to an embodiment.
FIG. 12 is a diagram illustrating an elastic member including a damper having parts that are bent in different directions for each part according to an embodiment.
FIG. 13 illustrates diagrams illustrating stress distribution according to structural analysis of an elastic member.
FIG. 14 is a diagram illustrating a vibration generating unit including a plurality of elastic members according to an embodiment.
FIG. 15 is a diagram illustrating a vibration generating unit including a plurality of elastic members according to an embodiment.
FIG. 16 is a block diagram for describing components constituting a vibration apparatus according to an embodiment.
FIG. 17 illustrates diagrams for describing heights of a vibration transmission module of a vibration apparatus.
FIG. 18 illustrates diagrams for describing heights of a vibration transmission module of a vibration apparatus.
FIG. 19 illustrates diagrams for describing heights of a vibration transmission module of a vibration apparatus.
FIG. 20 is a block diagram for describing a type of a vibration transmission module.
FIG. 21 illustrates diagrams for describing a type of a vibration transmission module and a relationship between the vibration transmission module and a vibration module.
FIG. 22 illustrates diagrams for describing a vibration transmission module according to another embodiment.
FIG. 23 is a block diagram for describing an application including a vibration apparatus.
FIG. 24 is a diagram for describing a vibration receiving unit of an application.

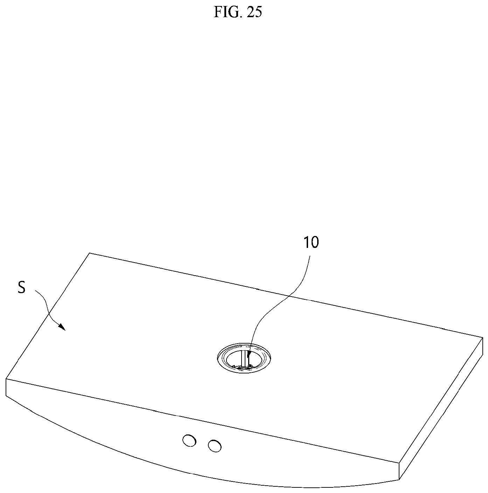

FIG. 25 is a diagram for describing a vibration receiving unit of an application.

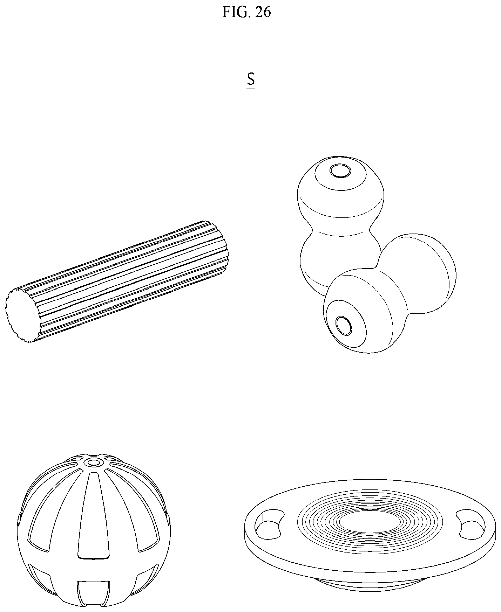

FIG. 26 is a diagram for describing a vibration receiving unit of an application.
FIG. 27 is a diagram for describing a vibration receiving unit of a massage device in which a vibration module is disposed.
FIG. 28 is a diagram for describing an exercise apparatus in which a vibration module is disposed.
FIG. 29 is a diagram for describing an arrangement direction of a vibration module disposed in a vibration receiving unit.
FIG. 30 is a diagram for describing an arrangement direction of a vibration module disposed in a vibration receiving unit.
FIG. 31 is a block diagram for describing additional functions provided by an application.
FIG. 32 is a diagram illustrating a vibration amplification unit for performing a cooling and heating function and a vibration function according to an embodiment.
FIG. 33 is a diagram illustrating a change in skin condition according to a vibration frequency according to an embodiment.

### DETAILED DESCRIPTION OF EXEMPLARY EMBODIMENTS

The above-described objects, features, and advantages of the present invention will be clear from the following detailed descriptions in connection with the accompanying drawings. However, while the present invention may have various modifications and alternative forms, specific embodiments thereof are shown by way of example in the drawings and will be described herein in detail.

The drawings attached to this specification are for easily explaining the present invention, and the shapes shown in the drawings may be exaggerated and displayed as necessary to aid in understanding of the present invention, and thus the present invention is not limited to the drawings.

When it is determined that detailed descriptions or configurations of related well-known functions may unnecessarily obscure the gist of the present invention, the detailed descriptions thereof will be omitted. Numbers (for example, first, second, etc.) used in the description of this specification are used only to identify one element from another element.

A suffix of "unit," "module," or "part" of an element used herein is assigned or incorporated for convenience of specification description and the suffix itself does not have a distinguished meaning or function.

In addition, components having the same function within the scope of the same idea illustrated in the drawing of each embodiment will be described using the same reference numerals.

According to an aspect of the present disclosure, there is provided an elastic member may comprise a body part, a first damper extending from a first point of the body part, and a second damper extending from a second point of the body part, wherein the first damper includes a first inner part extending in any one of a clockwise direction and a counter clockwise direction, based on a central axis of the body part, from the first point, a first connecting part extending in a U shape from the first inner part, and a first outer part extending in other one of the clockwise direction and the counter clockwise direction from the first connecting part, wherein the second damper includes a second inner part extending in any one of the clockwise direction and the counter clockwise direction, based on the central axis of the body part, from the second point, a second connecting part extending in a U shape from the second inner part, and a second outer part extending in other one of the clockwise direction and the counter clockwise direction from the second connecting part, and wherein the first damper and the second damper are apart from each other.

In some embodiments, wherein the first connecting part includes at least a first portion and a second portion with a different radius of curvature.

In some embodiments, wherein the first connecting part includes at least a first portion, a second portion and a third portion, having different radiuses of curvature and being arranged sequentially, and wherein a radius of curvature of the second portion is longer than a radius of curvature of the first portion or a radius of curvature of the third portion.

The drawings attached to this specification are for easily explaining the present invention, and the shapes shown in the drawings may be exaggerated and displayed as necessary to aid in understanding of the present invention, and thus the present invention is not limited to the drawings.

When it is determined that detailed descriptions or configurations of related well-known functions may unnecessarily obscure the gist of the present invention, the detailed descriptions thereof will be omitted. Numbers (for example, first, second, etc.) used in the description of this specification are used only to identify one element from another element.

A suffix of "unit," "module," or "part" of an element used herein is assigned or incorporated for convenience of specification description and the suffix itself does not have a distinguished meaning or function.

In addition, components having the same function within the scope of the same idea illustrated in the drawing of each embodiment will be described using the same reference numerals.

According to an aspect of the present disclosure, there is provided an elastic member may comprise a body part, a first damper extending from a first point of the body part, and a second damper extending from a second point of the body part, wherein the first damper includes a first inner part extending in any one of a clockwise direction and a counter clockwise direction, based on a central axis of the body part, from the first point, a first connecting part extending in a U shape from the first inner part, and a first outer part extending in other one of the clockwise direction and the counter clockwise direction from the first connecting part, wherein the transmission module configured to couple to the housing directly or indirectly and surround at least a part of the probe such that the vibration transmission module induces dispersion of external force to the probe, wherein the probe has a predetermined weight to amplify the vibration generated by the vibration generating unit.

In some embodiments, wherein, at least part of an upper part of the vibration transmission module is opened.

In some embodiments, wherein, the upper part of the vibration transmission module is closed.

In some embodiments, wherein a height of the vibration transmission module is set as a distance from a virtual plan perpendicular to a central axis of the bobbin to an end of the probe when the position of the bobbin is lowered to the maximum by the vibration of the bobbin is longer than a distance from the virtual plane to an end of the upper part of the vibration transmission module to induce dispersion of the external force applied to the probe to the vibration transmission module.

In some embodiments, wherein the height of the vibration transmission module is set as a distance from a virtual plan perpendicular to a central axis of the bobbin to an end of the probe when the position of the bobbin is raised to the maximum by the vibration of the bobbin is longer than a distance from the virtual plane to an end of the upper part of the vibration transmission module to transmit vibration indirectly by the probe.

In some embodiments, wherein the height of the vibration transmission module is set as a distance from a virtual plan perpendicular to a central axis of the bobbin to an end of the probe when the position of the bobbin is raised to the maximum by the vibration of the bobbin is shorter than a distance from the virtual plane to an end of the upper part of the vibration transmission module to transmit vibration directly by the probe.

In some embodiments, wherein the height of the vibration transmission module is set as a distance from a virtual plan perpendicular to a central axis of the bobbin to an end of the probe when the position of the bobbin is raised to the maximum by the vibration of the bobbin is longer than a distance from the virtual plane to an end of the upper part of the vibration transmission module to transmit vibration indirectly by the probe.

In some embodiments, wherein the vibration transmission module includes a first vibration transmission module formed to surround at least part of the probe and a second vibration transmission module formed to surround at least part of the housing,

In some embodiments, wherein the second vibration transmission module includes a plurality of coupling members capable of being coupled to at least part of the housing.

In some embodiments, wherein the housing includes a body part having an inner space and a connecting part formed on an outside of the body part to be coupled to the second vibration transmission module.

In some embodiments, wherein, the plurality of coupling members of the second vibration transmission module is formed in a cylindrical shape including a thread grove to be screwed with the connecting part of the housing.

In some embodiments, wherein the plurality of coupling members of the second vibration transmission module is coupled to the connecting part of the housing, and the second vibration transmission module is formed apart from the body part of the housing such that the vibration generated by the vibration module is transmitted to the second vibration transmission module.

In some embodiments, wherein the first vibration transmission module is connected to the second vibration transmission module, wherein the first vibration transmission module is formed apart from the vibration generating unit, and wherein the vibration generated by the vibration generating unit is transmitted to the first vibration transmission module through the second vibration transmission module.

In some embodiments, wherein the vibration transmission module is coupled to the housing such that the vibration generated by the vibration generating unit is transmitted to the vibration transmission module through the housing.

According to an aspect of the present disclosure, there is provided a massage device with acoustic wave, the massage device may comprise a vibration module comprising a vibration generating unit and a vibration amplification unit, wherein the vibration generating unit includes a housing providing an inner space, a magnet located in the housing, a bobbin located apart from the magnet in a direction of axis of the magnet, a coil disposed at an outer peripheral surface of the bobbin and an elastic member located on one side of the bobbin, and wherein the vibration amplification unit includes a probe connected to the bobbin and vibrating in the vertical direction, a vibration transmission module configured to couple to the housing directly or indirectly and surround at least a part of the probe such that the vibration transmission module induces dispersion of external force to the probe, and a vibration receiving unit configured to surround at least part of the vibration transmission module, wherein the probe has a predetermined weight to amplify the vibration generated by the vibration generating unit, wherein, the vibration transmission module is contacted to the vibration receiving unit such that the vibration amplified by the vibration amplification unit transmits to the vibration receiving unit, and wherein, the vibration receiving unit is not directly connected to the vibration amplification unit.

In some embodiments, wherein, the vibration receiving unit is a cushion-shaped.

In some embodiments, wherein, the vibration receiving unit includes at least one of the vibration modules, and wherein, the probe is placed in the vibration receiving unit so that the direction of vibration of the probe corresponds to the height direction of the vibration receiving unit.

In some embodiments, wherein, the massage device comprises a cover, wherein the cover surrounds at least one of the vibration receiving unit or the vibration transmission module.

According to an aspect of the present disclosure, there is provided an exercise apparatus using acoustic wave, the exercise apparatus may comprise a massage device with acoustic wave, the massage device comprising a vibration module comprising a vibration generating unit and a vibration amplification unit, wherein the vibration generating unit includes a housing providing an inner space, a magnet located in the housing, a bobbin located apart from the magnet in a direction of axis of the magnet, a coil disposed at an outer peripheral surface of the bobbin and an elastic member located on one side of the bobbin, and wherein the vibration amplification unit includes a probe connected to the bobbin and vibrating in the vertical direction, a vibration transmission module configured to couple to the housing directly or indirectly and surround at least a part of the probe such that the vibration transmission module induces dispersion of external force to the probe, and a vibration receiving unit configured to surround at least part of the vibration transmission module, wherein the probe has a predetermined weight to amplify the vibration generated by the vibration generating unit, wherein, the vibration transmission module is contacted to the vibration receiving unit such that the vibration amplified by the vibration amplification unit transmits to the vibration receiving unit, wherein, the vibration receiving unit is not directly connected to the vibration amplification unit, and wherein, the vibration transmission module is located inside the vibration receiving unit.

In some embodiments, wherein, wherein the exercise apparatus comprises a connecting shaft that penetrates the elastic member, the bobbin, the magnet and the housing, wherein, the connecting shaft further comprises the probe vibrating in a vertical direction corresponding to the bobbin at the lower part of the connecting shaft.

In some embodiments, wherein, wherein, the probe is formed so that a plurality of wires of an elastic material are radially spread.

In some embodiments, wherein, wherein, the vibration receiving unit is formed in a spherical shape.

In some embodiments, wherein, wherein, the vibration receiving unit is formed in a cylindrical shape.

In some embodiments, wherein, wherein, the vibration module is placed in the vibration receiving unit so that a vibration direction of the probe is perpendicular to the height direction of the vibration receiving unit.

In some embodiments, wherein, wherein, the vibration module is placed in the vibration receiving unit so that a vibration direction of the probe is parallel to the height direction of the vibration receiving unit.

In some embodiments, wherein, wherein, the material of the vibration receiving unit is EVA(Ethylene Vinyl Acetate Copolymer) or EPP(Expanded Polypropylene).

Hereinafter, acoustic wave vibration apparatuses according to embodiments will be described with reference to the accompanying drawings.

Embodiments of the present invention relate to a vibration module 1000 for transmitting acoustic wave vibration to the human body, and an application 20 in which the vibration module 1000 is mounted, and more specifically, to a vibration module 1000 that can provide vibration generated based on acoustic pressure for efficient vibration transmission, and an application 20 in which the vibration module 1000 is mounted. In a vibration module 1000 according to an embodiment, a probe 1210 having an appropriate weight may be connected to a component that generates acoustic pressure so that strong vibration is generated in the probe 1210, and the vibration generated by the vibration module 1000 may be indirectly transmitted to the human body through a vibration transmission module 200 connected to the vibration module 1000 so that waves of acoustic waves that are beneficial may be smoothly and deeply transmitted to the human body. An application 20 in which the vibration module 1000 is mounted may provide natural and comfortable vibration to the human body.

The acoustic wave vibration refers to waves generated when a medium such as air or water receives vibration of a sounding body. That is, the acoustic wave vibration refers to vibration generated due to acoustic pressure. In this case, the vibration generated due to the acoustic pressure may refer to vibration generated when sound is transmitted using a liquid, gas, or solid as a medium or may refer to vibration of a mechanical structure itself generated by movement of the mechanical structure (e.g., a cone paper damper, a leaf spring, an elastic member, etc.) that generates sound.

Meanwhile, as a frequency range applied to acoustic wave vibration according to an embodiment, an audible frequency band that is safe to the human body and that can provide various positive effects may be used. That is, when sound in a range of 20 Hz or lower is defined as infrasound, sound in a range of 20 Hz to 20 KHz is defined as acoustic sound, and sound in a range of 20 KHz or higher is defined as ultrasound, the vibration module 1000 according to the embodiment of the present invention may output acoustic wave in a range of about 20 Hz to 20 KHz, which is an audible frequency band, as acoustic vibration to enable more effective and safe stimulation and massage to be performed on the human body.

A frequency applied to acoustic wave vibration according to an embodiment may be in a range of 80 to 300 Hz. For example, the frequency range may include a frequency in an octave relationship with 440 Hz (a note A), which is preferred in the field of healing using music, that is, a frequency of 110 Hz, which is 1/4 of 440 Hz (a note A), or a frequency of 220 Hz, which is 1/2 of 440 Hz (a note A).

An acoustic wave vibration apparatus 100 according to an embodiment may include a vibration module 1000 and a control module 2000.

The vibration module 1000 may include components such as a housing 1170, a bracket 1150, a magnet 1110, a bobbin 1120, a coil 1130, an elastic member 1140, and a probe 1210, which will be described below, and may be driven by receiving power from a power supply.

Meanwhile, the control module 2000 may be a component for controlling the vibration module 1000 and may include an input unit 2300, an output unit 2400, a communication unit 2200, and a controller 2100 and adjust an input of a sound source having a specific frequency and an amplitude of the sound source.

Hereinafter, various embodiments of the vibration module 1000 will be described. However, the present invention is not limited to the contents described in each embodiment and, in addition to the contents specified as the embodiments of the vibration module 1000, embodiments in which various components to be described below are combined may also be included in the present invention.

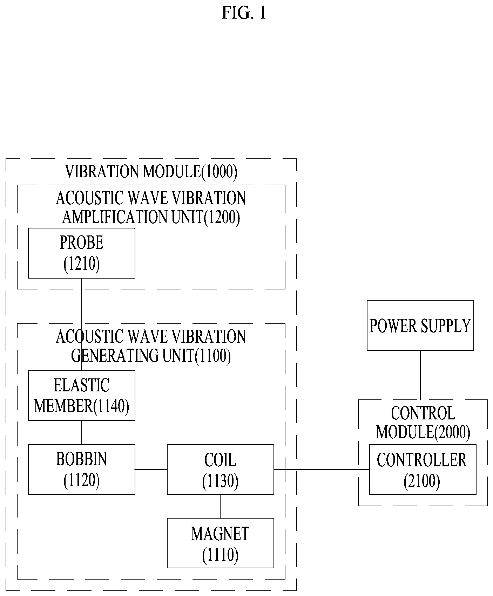

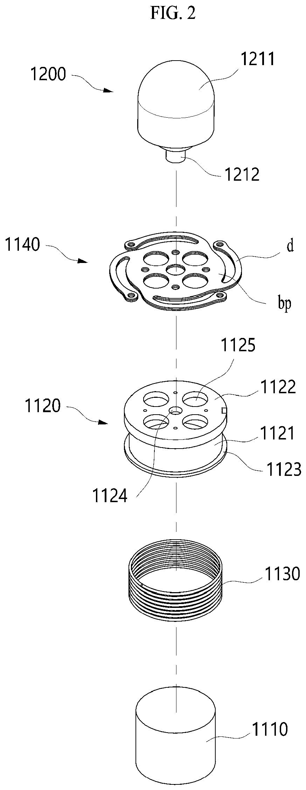

FIG. 1 is a block diagram for describing components constituting an acoustic wave vibration apparatus according to a first embodiment, and FIG. 2 is a diagram for describing components constituting a vibration module according to the first embodiment. Hereinafter, the vibration module according to the first embodiment will be described with reference to FIGS. 1 and 2.

Referring to FIGS. 1 and 2, the vibration module 1000 according to the first embodiment may include an acoustic wave vibration generating unit 1100 and an acoustic wave vibration amplification unit 1200. In this case, the acoustic wave vibration generating unit 1100 of the vibration module 1000 according to the first embodiment may include a magnet 1110, a bobbin 1120, a coil 1130, and an elastic member 1140, and the acoustic wave vibration amplification unit 1200 of the vibration module 1000 according to the first embodiment of the present invention may include a probe 1210.

Hereinafter, each of configurations of the acoustic wave vibration generating unit 1100 and the acoustic wave vibration amplification unit 1200 will be described in detail with reference to the drawings.

The vibration module 1000 according to the first embodiment of the present invention includes the magnet 1110.

The magnet 1110 may be located inside a housing 1170 or a lower bracket 1150a, which will be described below, and may be detachably provided to a fixing groove formed in the housing 1170.

Although not illustrated in FIG. 2, in some embodiments, the magnet 1110 may be formed as an F-type magnet surrounding the coil 1130.

The magnet 1110 may generate vibration by interacting with the coil 1130 which will be described below. That is, the vibration module 1000 may vibrate according to a force induced due to an interaction between a current flowing through the coil 1130 and a magnetic field generated by the magnet 1110. In this case, the magnet 1110 may be spaced a predetermined interval from an inner surface of the housing 1170 to be fixedly provided inside the housing 1170, and thus a space between the magnet 1110 and the inner surface of the housing 1170 may provide a path for the magnetic field generated by the magnet 1110.

The magnet 1110 may have magnetic pole surfaces of N and S poles, and the magnet 1110 may be disposed in the housing 1170 so that the S pole of the magnet 1110 faces a bottom surface of the housing 1170, but the present invention is not limited thereto. As another example, the magnet 1110 may be disposed in the housing 1170 so that the N pole of the magnet 1110 faces the bottom surface of the housing 1170.

The magnet 1110 may be formed to have a shape corresponding to a shape of the housing 1170. More specifically, the shape of the magnet 1110 may correspond to a shape of the fixing groove provided in the housing 1170. For example, the magnet 1110 may have a cylindrical shape. As another example, the magnet 1110 may have a cylindrical shape having a diameter of 25 mm and a thickness of 20 T.

Although not illustrated in FIG. 2, a silicon pad or the like may be provided on a bottom surface of the magnet 1110 to prevent loss of magnetic force. Accordingly, it is possible to minimize the occurrence of loss of magnetic force using a cover with which the bottom surface of the magnet 1110 is brought into contact.

The magnet 1110 may be provided as a permanent magnet made of a ferromagnetic material, such as a neodymium magnet, but the present invention is not limited thereto.

The vibration module 1000 according to the first embodiment of the present invention includes the bobbin 1120.

The bobbin 1120 may be a component for winding the coil 1130, which will be described below, and when a magnetic force is generated due to an interaction between the magnet 1110 and the coil 1130, the bobbin 1120 may be moved by the magnetic force.

Specifically, at least a part of the bobbin 1120 may be disposed in the housing 1170. More specifically, the at least a part of the bobbin 1120 may be disposed in the housing 1170 and may be disposed between the inner surface of the housing 1170 and the magnet 1110. In this case, the at least a part of the bobbin 1120 may be disposed to be spaced apart from the inner surface of the housing 1170 and the magnet 1110 without being in contact with the inner surface of the housing 1170 and the magnet 1110. For example, when the bobbin 1120 vibrates up and down due to the interaction between the magnet 1110 and the coil 1130, at least a part of the bobbin 1120 may be disposed to be spaced apart from the inner surface of the housing 1170 and the magnet 1110 without being in contact with the inner surface of the housing 1170 and the magnet 1110 such that the bobbin 1120 does not interfere with the housing 1170 and the magnet 1110.

Further, in another embodiment, the bobbin 1120 may be disposed above the magnet 1110.

The bobbin 1120 may include a cylindrical main body part 1121 having open upper and lower parts and an inner space, an upper surface 1122 formed on an upper part of the main body part 1121, and a lower surface 1123 formed on a lower part of the main body part 1121.

The at least a part of the magnet 1110 described above may be disposed in the inner space of the main body part 1121. For example, a part corresponding to 1/4 of the thickness of the magnet 1110 may be disposed in the inner space of the main body part 1121 of the bobbin 1120. Further, the inner space of the main body part 1121 may be provided as a space such that the magnet 1110 can be spaced a predetermined interval from the inner surface of the main body part 1121. That is, a radius of the inner space of the main body part 1121 may be greater than a radius of the magnet 1110.

The coil 1130 which will be described below may be provided in at least a part of a side surface of the main body part 1121. That is, the coil 1130 may be provided along an outer peripheral surface of the main body part 1121. For example, the coil 1130 may be wound around an entirety of the side surfaces of the main body part 1121. As another example, the coil 1130 may be overlapped at least once on the side surfaces of the main body part 1121. As a result, when the coil 1130 receives a current and then vibrates up and down due to the interaction with the magnet 1110, the bobbin 1120 may also vibrate up and down together with the coil 1130.

Meanwhile, the upper surface 1122 of the bobbin 1120 may be provided to have a plate shape in which at least a part thereof is open. That is, the at least a part of the upper surface 1122 of the bobbin 1120 may have an opening, and the opening may be a coupling hole 1124 or a heat dissipation hole 1125.

The coupling hole 1124 may be formed in the center of the upper surface 1122 of the bobbin 1120. The probe 1210 which will be described below may be coupled to the bobbin 1120 through the coupling hole 1124 formed in the upper surface 1122 of the bobbin 1120. In this case, the probe 1210 may be coupled to the bobbin 1120 through the coupling hole 1124 of the upper surface 1122 using a screw coupling method, but the present invention is not limited thereto. Meanwhile, in order to minimize eccentricity that may occur in the vibration module 1000 when the human body is stimulated according to the vibration of the vibration module 1000, the coupling hole 1124 may be formed in the center of the upper surface 1122 of the bobbin 1120.

At least one heat dissipation hole 1125 may be provided in the upper surface 1122 of the bobbin 1120. Further, the heat dissipation hole 1125 may be provided in an outside of the coupling hole 1124. At least one heat dissipation hole 1125 may be formed in the upper surface 1122 of the bobbin 1120, and thus heat generated from the coil 1130 may be discharged to the outside through the heat dissipation hole 1125 and noise generated during vibration may also be reduced. More specifically, when the vibration module 1000 is continuously operated, a great deal of heat may be generated in the region around the coil 1130, which may cause discomfort when using the vibration module 1000 or cause degradation of the performance of the vibration module 1000. As a result, the heat generated in the region around the coil 1130 may be discharged through the heat dissipation hole 1125 provided in the bobbin 1120, thereby solving the above problem.

An outer diameter of the upper surface 1122 of the bobbin 1120 may be greater than an outer diameter of the main body part 1121. That is, a radius of the upper surface 1122 may be greater than a radius of the main body part 1121. In other words, the upper surface 1122 may be formed to cover an upper part of the main body part 1121. More specifically, at least some of outer regions of the upper surface 1122 may protrude outward from the main body part 1121. In this case, a degree of protrusion of the outer regions of the upper surface 1122 outward from the main body part 1121 may be greater than or equal to a thickness of at least one coil 1130. For example, at least some of the outer regions of the upper surface 1122 may be formed to protrude outward from the main body part 1121 so that the coil 1130 provided on the side surface of the main body part 1121 cannot deviate to the outside due to the upper surface 1122 despite the vibration of the vibration module 1000.

The lower surface 1123 of the bobbin 1120 may be formed by extending at least some of lower regions of the main body part 1121 to the outside. That is, the lower surface 1123 of the bobbin 1120 may be formed to have a protruding annular frame shape in which at least some of the lower regions of the main body part 1121 protrude outward. In this case, a degree of protrusion of the lower surface 1123 of the bobbin 1120 may correspond to the degree of protrusion of the upper surface 1122 outward from the main body part 1121. Accordingly, as described above, the coil 1130 provided on the side surface of the main body part 1121 may be guided by the protruding parts of the upper surface 1122 and the lower surface 1123 and may not deviate to the outside despite the vibration of the vibration module 1000.

The vibration module 1000 according to the first embodiment of the present invention includes the coil 1130.

As described above, the coil 1130 may be provided in at least a part of the side surface of the main body part 1121 of the bobbin 1120.

When a current is applied to the coil 1130, the coil 1130 may vibrate up and down through an interaction with the magnet 1110. In this case, in order to increase an intensity of the vibration of the vibration module 1000, the coil 1130 having a large thickness may be used or the coil 1130 may be provided to be wound around the bobbin 1120 several times. For example, the coil 1130 may be provided in two or more layers in all regions of the side surfaces of the main body part 1121 of the bobbin 1120.

Meanwhile, as described above, the coil 1130 may be guided by the protruding parts of the upper surface 1122 and the lower surface 1123 and may not deviate to the outside despite the vibration of the vibration module 1000.

The vibration module 1000 according to the first embodiment of the present invention includes the elastic member 1140.

The elastic member 1140 may include a plate-shaped body part bp and a damper d extending from the body part bp.

The elastic member 1140 may include the plate-shaped body part bp. The body part bp of the elastic member 1140 may have a flat plate shape, but the present invention is not limited thereto. For example, the shape of the body part bp may be a curved shape bent to have a predetermined curvature. As another example, the body part bp of the elastic member 1140 may be provided to have a circular plate shape and may be formed to correspond to a size and shape of the upper surface 1122 of the bobbin 1120.

The elastic member 1140 may be provided on the upper surface 1122 of the bobbin 1120. More specifically, the body part bp of the elastic member 1140 may be provided on the upper surface 1122 of the bobbin 1120. That is, in the body part bp, a plurality of coupling holes that can be coupled to the upper surface 1122 of the bobbin 1120 may be provided, and the elastic member 1140 may be coupled to the upper surface 1122 of the bobbin 1120 through the plurality of coupling holes. In this case, the coupling method may be a screw coupling method, but the present invention is not limited thereto.

Further, the body part bp of the elastic member 1140 may be coupled to the probe 1210. In a vibration module 1000 according to an embodiment, the coupling hole that can be coupled to the probe 1210 may be additionally formed in the center of the body part bp, and the elastic member 1140 may be screwed to the probe 1210 through the coupling hole formed in the center of the body part bp. In a vibration module 1000 according to another embodiment, the probe 1210 may be detachably coupled to the body part bp by a magnetic material formed in the center of the body part bp. Accordingly, the elastic member 1140 may transmit vibration generated due to an interaction between the coil 1130 and the magnet 1110 to the probe 1210.

The elastic member 1140 may include a damper d extending from the body part bp. At least one damper d may be provided.

The damper d of the elastic member 1140 may be formed to extend along an outer peripheral surface of the body part bp. That is, at least a part of the outer peripheral surface of the body part bp may be formed to extend to the outside and may be formed to extend in a circumferential direction of the body part bp.

In an embodiment, the damper d may be provided to have a curved shape. For example, a plurality of dampers d may be formed and may be implemented to have curved shapes having different radii of curvature.

For example, the damper d may be provided to have a shape extending in a radially curved shape of the body part bp. Further, at least two dampers d may be formed to extend along the outer peripheral surface of the body part bp. As another example, the damper d may be formed to have a U shape or an S shape.

The damper d may be fixedly provided on an upper part of the lower bracket 1150a or the housing 1170. More specifically, the damper d may include a fixing part 1145 at an end thereof and may be fixedly provided on the upper part of the lower bracket 1150a or the housing 1170 through the fixing part 1145.

In this case, the fixing part 1145 may be fixed to the vibration module 1000 in various ways. For example, the damper d may include a coupling hole. In this case, a coupling protrusion 1151a may be included on an upper part of the housing 1170 or an upper surface of the lower bracket 1150a, and the coupling protrusion 1151a may include a coupling hole. The elastic member 1140 may be fixed to the upper part of the housing 1170 or the upper surface of the lower bracket 1150a through the coupling hole of the fixing part 1145 and the coupling hole of the coupling protrusion 1151a. In this case, the coupling method may be a screw coupling method, but the present invention is not limited thereto.

As another example, the fixing part 1145 may be adhesively coupled to the housing 1170 or the lower bracket 1150a. As still another example, in an inside of the housing 1170 or an inside of the lower bracket 1150a, a connecting part for connecting the damper d may be included, and the fixing part 1145 and the connecting part may be coupled in a fitting form. The method of fixedly providing the damper d and the connecting part is not limited thereto, and the damper d and the connecting part may be fixedly provided in various forms.

Hereinafter, an operation of the elastic member 1140 will be described.

In a vibration module 1000 according to an embodiment, when a current is applied to the coil 1130 and then an elastic force is generated from the elastic member 1140 according to a force induced due to the interaction between the coil 1130 and the magnet 1110, vibration may be generated.

The elastic member 1140 may generate vibration in a vertical direction according to the force induced due to the interaction between the current flowing through the coil 1130 and the magnetic field generated by the magnet 1110. That is, the elastic member 1140 may generate vibration in response to a sound source supplied from the outside.

More specifically, the body part bp of the elastic member 1140 may be coupled to the bobbin 1120 so that, when the bobbin 1120 is vertically moved according to the force induced due to the interaction between the coil 1130 and the magnet 1110, the elastic member 1140 may also be vertically moved, and the damper d of the elastic member 1140 may be coupled to the lower bracket 1150a so that the vertical movement may be performed with a predetermined amplitude and the elastic member 1140 may vibrate.

As a result, the body part bp of the elastic member 1140 may be coupled to the upper surface 1122 of the bobbin 1120 so that, when the bobbin 1120 is vertically moved due to the interaction between the coil 1130 and the magnet 1110, the damper d of the elastic member 1140 may be fixed and the body part bp of the elastic member 1140 may be coupled to the bobbin 1120, and, accordingly, the elastic force may be generated according to the movement of the bobbin 1120 by the elastic member 1140. Due to the generation of the elastic force, the elastic member 1140 may vibrate together with the bobbin 1120.

The damper d of the elastic member 1140 may guide the bobbin 1120 to vertically move within a predetermined range.

More specifically, when a current is applied to the coil 1130 and then the bobbin 1120 is vertically moved by the force induced due to the interaction between the coil 1130 and the magnet 1110, the damper d may guide the bobbin 1120 to vertically move within a predetermined range. That is, the damper d may enable the coil 1130, the bobbin 1120, or the probe 1210 to vibrate with a predetermined amplitude when the vibration module 1000 vibrates. More specifically, when an upward force is generated due to the interaction between the coil 1130 and the magnet 1110, the bobbin 1120 is moved upward, and the damper d may guide the bobbin 1120 so as to continue to move upward and not to deviate to the outside of the vibration module.

The elastic member 1140 may minimize the horizontal movement of the bobbin 1120 when the vibration module 1000 vibrates. That is, the elastic member 1140 may perform a function in which a tilting phenomenon is prevented from occurring in the bobbin 1120 when the vibration module 1000 vibrates. More specifically, the vibration module 1000 performs a massage function so that eccentricity may occur in the vibration module 1000 due to a physical force generated from the outside, and thus the vibration module 1000 according to the embodiment may minimize the eccentricity problem by coupling the elastic member 1140 to the lower bracket 1150a and the bobbin 1120.

At least one heat dissipation hole may be formed in the body part bp of the elastic member 1140. In this case, the heat dissipation holes may be provided between the plurality of coupling holes. The heat dissipation holes formed in the body part bp may be formed to correspond to a position, size, and shape of the heat dissipation hole 1125 formed on the upper surface 1122 of the bobbin 1120 described above. In this case, since the function, action, and effect of the heat dissipation hole formed in the elastic member 1140 are the same as those of the heat dissipation hole 1125 formed in the upper surface 1122 of the bobbin 1120 described above, detailed descriptions thereof will be omitted.

When there are a plurality of dampers d of the elastic member 1140 and the plurality of dampers d are disposed at regular intervals, an elastic force generated from each of the plurality of dampers d may be applied to a central part of the elastic member 1140 with the same magnitude.

An elastic member 1140 of a vibration module 1000 according to another embodiment may have a plurality of dampers d. That is, when the elastic member 1140 has the plurality of dampers d and the plurality of dampers d are disposed at regular intervals, an elastic force generated from each of the plurality of dampers d may be applied to a central part of the elastic member 1140 with the same magnitude. Accordingly, the probe 1210 may be coupled to the central part of the elastic member 1140 so that eccentricity due to the elastic force generated from each of the plurality of dampers d may be prevented.

A vibration module 1000 according to still another embodiment may include a plurality of elastic members 1140. That is, two or more of the above-described elastic members 1140 may be disposed in the vibration module 1000, and thus it is possible to further increase the elastic force to induce strong vibration and, at the same time, it is possible to provide an effect of maintaining durability even with strong vibration.

The vibration module 1000 according to the first embodiment of the present invention includes the probe 1210. The probe 1210 may include a head part 1211 and a connecting part 1212.

The head part 1211 of the probe 1210 may be provided to have a predetermined weight. That is, the head part 1211 may have a certain weight for maximizing vibration efficiency of the vibration module 1000.

More specifically, in the case in which a magnetic field with the same magnitude is formed in the vibration module 1000, even when the magnetic force generated due to the interaction between the coil 1130 and the magnet 1110 is the same, a magnitude of vibration generated by the elastic member 1140 when the probe 1210 having an appropriate weight is coupled to the elastic member 1140 may be greater than a magnitude of vibration generated by the elastic member 1140 when the probe 1210 is not coupled to the elastic member 1140.

Therefore, the probe 1210 having a certain weight for maximizing the magnitude of the vibration generated by the elastic member 1140 may be coupled to the elastic member 1140, and thus the vibration efficiency of the vibration module 1000 may be maximized.

The head part 1211 of the probe 1210 may be a component which is brought into direct contact with the human body, but the present invention is not limited thereto. For example, the head part 1211 may not be brought into contact with the human body and may only serve to maximize the vibration efficiency.

Further, the connecting part 1212 of the probe 1210 may be coupled to at least one of the bobbin 1120 and the elastic member 1140. For example, as described above, the connecting part 1212 of the probe 1210 may be coupled to the bobbin 1120 and the elastic member 1140 through the coupling hole 1124 formed in the upper surface 1122 of the bobbin 1120 and the coupling hole formed in the center of the body part bp of the elastic member 1140. Accordingly, the probe 1210 may be coupled to the bobbin 1120 and the elastic member 1140 and thus may vibrate up and down together with the bobbin 1120 and the elastic member 1140 by the force generated due to the interaction between the coil 1130 and the magnet 1110. Further, according to the arrangement or connection method of the bobbin 1120 and the elastic member 1140, the connecting part 1212 of the probe 1210 may be coupled only to the bobbin 1120 or may be coupled only to the elastic member 1140.

Further, the connecting part 1212 of the probe 1210 and the bobbin 1120 and the elastic member 1140 may be coupled in a screw coupling method, but the present invention is not limited thereto. For example, the connecting part 1212 of the probe 1210 may be provided in the form of a bolt that can be screwed to the bobbin 1120 and the elastic member 1140.

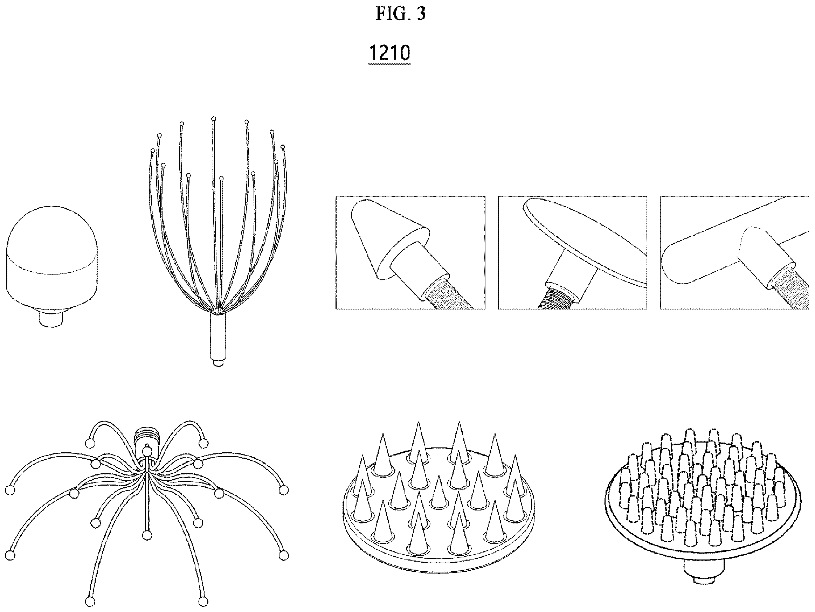

FIG. 3 is a diagram for describing an example of a shape of a head part of a probe according to an embodiment.

Referring to FIG. 3, the head part 1211 of the probe 1210 may be provided to have various shapes. According to an embodiment, when the head part 1211 is brought into contact with the human body, the head part 1211 may be provided to have a shape suitable for performing a function of massaging the human body. For example, when the head part 1211 performs a function of intensively stimulating a specific part, an end of the head part 1211 may have a sharp shape. As another example, when the head part 1211 performs a function of uniformly massaging a part such as a face or the like, the end of the head part 1211 may have a flat shape having a large area. As still another example, when the head part 1211 performs a function of massaging a scalp, the head part 1211 may have a shape in which a plurality of wires radially spread. In addition to the shapes of the head part 1211, the head part 1211 according to the embodiment may have a shape suitable for performing various massaging functions, or may have various known shapes of the head part 1211.

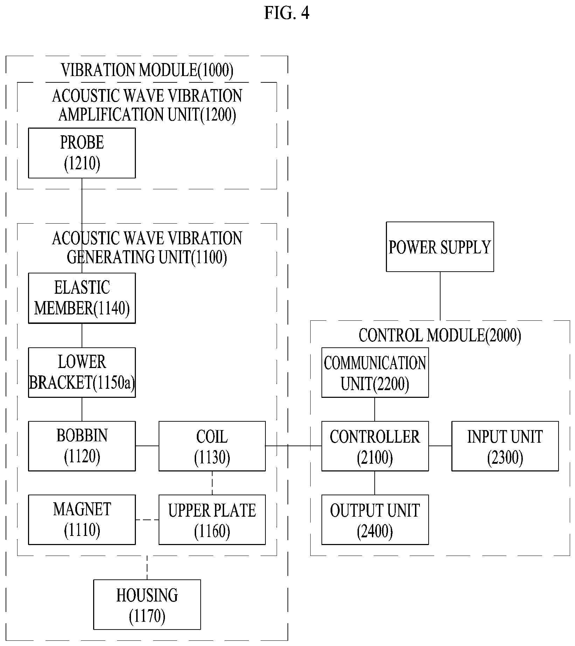

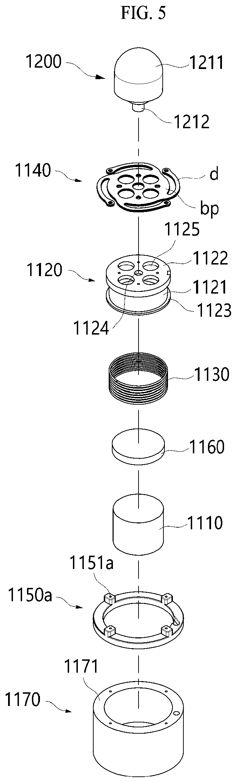

FIG. 4 is a block diagram for describing components constituting an acoustic wave vibration apparatus according to a second embodiment, and FIG. 5 is a diagram for describing components constituting a vibration module according to the second embodiment. Hereinafter, the vibration module according to the second embodiment will be described with reference to FIGS. 4 and 5.

Referring to FIGS. 4 and 5, a vibration module 1000 according to the second embodiment may include an acoustic wave vibration generating unit 1100, an acoustic wave vibration amplification unit 1200, and a housing 1170. In this case, the acoustic wave vibration generating unit 1100 of the vibration module 1000 according to the second embodiment of the present invention may include a magnet 1110, an upper plate 1160, a coil 1130, a bobbin 1120, a lower bracket 1150a, and an elastic member 1140, and the acoustic wave vibration amplification unit 1200 of the vibration module 1000 according to the second embodiment of the present invention may include a probe 1210.

Meanwhile, the magnet 1110, the bobbin 1120, the coil 1130, the elastic member 1140, and the probe 1210 of the vibration module 1000 according to the second embodiment correspond to the magnet 1110, the bobbin 1120, the coil 1130, the elastic member 1140, and the probe 1210 of the vibration module 1000 according to the first embodiment, respectively, and contents thereof are described above, and thus the same reference numerals are assigned to common parts, and detailed descriptions thereof will be omitted.

Hereinafter, the lower bracket 1150a, the upper plate 1160, and the housing 1170, which are components of the vibration module 1000 according to the second embodiment, will be described in detail with reference to the drawings.

The vibration module 1000 according to the second embodiment of the present invention may include the lower bracket 1150a provided to have a cylindrical shape having open upper and lower parts.

The lower bracket 1150a may include an upper surface that can be coupled to the elastic member 1140 described above and a lower surface that can be coupled to the housing 1170 which will be described below.

In this case, a plurality of coupling protrusions 1151a may be provided on the upper surface of the lower bracket 1150a, and the plurality of coupling protrusions 1151a may be formed to have a shape protruding by a predetermined distance or more from the upper surface of the lower bracket 1150a. Further, coupling holes through which the elastic member 1140, which will be described below, may be coupled may be provided in the plurality of coupling protrusions 1151a formed on the upper surface of the lower bracket 1150a. Further, the coupling holes provided in the plurality of coupling protrusions 1151a may be formed to pass through the lower bracket 1150a.

The coupling holes provided in the plurality of coupling protrusions 1151a may be formed to have a position, number, size, or shape corresponding to those of a plurality of coupling holes 1171 provided on an upper surface of the housing 1170 which will be described below. In other words, the plurality of coupling protrusions 1151a may be formed to protrude at a position of the upper surface of the lower bracket 1150a corresponding to the position of the coupling hole 1171 formed on the upper surface of the housing 1170. More specifically, the coupling holes provided in the plurality of coupling protrusions 1151a of the lower bracket 1150a may be formed to be connected to the plurality of coupling holes 1171 formed on the upper surface of the housing 1170.

Meanwhile, the upper surface of the lower bracket 1150a and the elastic member 1140 may be coupled in a screw coupling method and the lower surface of the lower bracket 1150a and the housing 1170 may be coupled in a screw coupling method, but the present invention is not limited thereto. For example, the upper surface of the lower bracket 1150a and the elastic member 1140 may be coupled and the lower surface of the lower bracket 1150a and the housing 1170 may be coupled so that durability may be secured and a vibration force may be maintained when the vibration module 1000 vibrates. Further, the lower bracket 1150a may include a silicone washer. The silicone washer may be provided on the lower surface of the lower bracket 1150a or the coupling protrusion 1151a and may serve as a buffer.

The plurality of coupling protrusions 1151a may be provided to have a shape protruding by a predetermined distance or more from the upper surface of the lower bracket 1150a. In this case, when the elastic member 1140 vibrates according to the operation of the vibration module 1000, the plurality of coupling protrusions 1151a may be formed so that the protruding height corresponds to a maximum amplitude of the elastic member 1140.

More specifically, when the elastic member 1140 vibrates according to the operation of the vibration module 1000, the plurality of coupling protrusions 1151a may be formed to protrude from the upper surface of the lower bracket 1150a so that interference does not occur between the elastic member 1140 and the upper surface of the lower bracket 1150a. For example, the protruding heights of the plurality of coupling protrusions 1151a may be less than or equal to the maximum amplitude of the elastic member 1140, but the present invention is not limited thereto. As another example, when the maximum amplitude of the elastic member 1140 is 3 mm, the protruding heights of the plurality of coupling protrusions 1151a may be 3 mm or more.

Further, the protruding heights of the plurality of coupling protrusions 1151a may be formed in consideration of a maximum vibration amplitude of the vibration module 1000, an inner depth of the housing 1170, a height of the lower bracket 1150a, and a height of the bobbin 1120. That is, the plurality of coupling protrusions 1151a may have the protruding height so that the bobbin 1120 may not be brought into contact with the housing 1170 when the vibration module 1000 vibrates.

The vibration module 1000 according to the second embodiment of the present invention may include the upper plate 1160. The upper plate 1160 may be provided on the upper surface of the magnet 1110 or may be disposed adjacent to the lower surface of the bobbin 1120.

The upper plate 1160 may be provided on the upper surface of the magnet 1110 so that the upper plate 1160 may serve to guide a magnetic field generated by the magnet 1110 to be concentrated on the coil 1130. That is, when the magnetic field is formed from an N pole to an S pole of the magnet 1110, the upper plate 1160 may serve to guide a magnetic path of the formed magnetic field to be formed more intensively in the coil 1130. More specifically, since the upper plate 1160 is provided on the upper surface of the magnet 1110, the magnetic field may be further formed in a direction toward the coil 1130 than in a direction toward the bobbin 1120 from the upper surface of the magnet 1110 as compared to the case in which the upper plate 1160 is not provided on the upper surface of the magnet 1110. As a result, the upper plate 1160 may be provided and thus loss of the magnetic field generated by the magnet 1110 may be prevented.

The upper plate 1160 may have a shape similar to the shape of the upper surface of the magnet 1110. More specifically, the upper plate 1160 may have the same shape and size as the upper surface of the magnet 1110. Alternatively, the upper plate 1160 may have the same shape as the upper surface of the magnet 1110 but may have a large size at a certain ratio. For example, the upper plate 1160 may have a size and shape sufficient to cover an entirety of the upper surface of the magnet 1110.

The vibration module 1000 according to the second embodiment of the present invention includes the housing 1170 forming an inner space. The inner space may provide a space for accommodating the magnet 1110, the bobbin 1120, the coil 1130, and the like described above.

Further, the housing 1170 may be provided to have a cylindrical shape having an open upper part, but the present invention is not limited thereto. For example, at least a part of a bottom surface of the housing 1170 may include an open part.

More specifically, the magnet 1110 may be fixedly provided on a bottom surface of the inner space of the housing 1170. For example, the magnet 1110 may be disposed in the inner space of the housing 1170 so that a central point of the bottom surface of the inner space coincides with a central point of the bottom surface of the magnet 1110.

Further, a fixing groove in which the magnet 1110 may be fixedly provided may be formed in the bottom surface of the inner space of the housing 1170. The fixing groove may be formed to correspond to the size and shape of the magnet 1110, and the fixing groove may be formed to have a depth so that the magnet 1110 cannot deviate to the outside of the fixing groove due to the vibration of the vibration module 1000 after the magnet 1110 is inserted and disposed. Further, the fixing groove may be formed of a metal material so that the magnet 1110 is strongly coupled to the fixing groove and cannot deviate to the outside and is fixed despite the vibration of the vibration module 1000, but the present invention is not limited thereto.

Further, the fixing groove may be formed so that an outer peripheral surface of the magnet 1110 fixed to the fixing groove may be spaced a predetermined interval from the housing 1170, and may provide a magnetic path of the magnetic field formed by the magnet 1110 and the coil 1130.

Further, the plurality of coupling holes 1171 in which the above-described lower bracket 1150a may be mounted may be provided in the upper surface of the housing 1170. In this case, the lower bracket 1150a may be mounted on the upper surface of the housing 1170 in a screw coupling method, but the present invention is not limited thereto, and the lower bracket 1150a may be mounted using various known coupling methods.

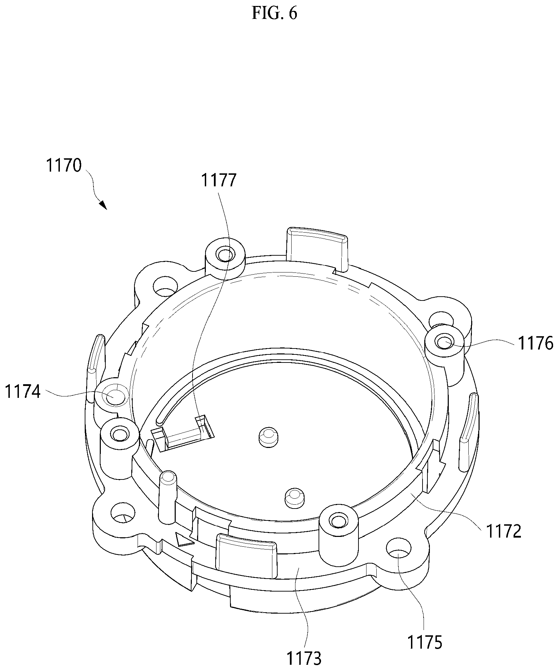

FIG. 6 is a diagram for describing a housing of a vibration module according to another embodiment.

Referring to FIG. 6, a housing 1170 of a vibration module 1000 according to another embodiment may include a body part 1172 and a connecting part 1173.

That is, the housing 1170 of the vibration module 1000 according to another embodiment may include the body part 1172 having an inner space, and the connecting part 1173 formed on an outer peripheral surface of the body part 1172.

The body part 1172 of the housing 1170 may provide a space for accommodating the magnet 1110, the bobbin 1120, the coil 1130, and the like, as described above, and a detailed description thereof will be omitted since the above components have been described above.

Further, the body part 1172 of the housing 1170 may include a wire through-hole 1174. The wire through-hole 1174 may provide a space through which a wire can pass. The wire through-hole 1174 may provide a space through which a wire connected to the coil 1130 can exit toward a lower surface of the housing 1170 in order to apply power to the coil 1130.

The connecting part 1173 of the housing 1170 may be formed to protrude from an outer peripheral surface of at least one body part 1172. Further, the connecting part 1173 of the housing 1170 may include at least one coupling hole 1175. In this case, the at least one coupling hole 1175 may be coupled to a vibration transmission module 200 which will be described below. Detailed descriptions of the coupling of the connecting part 1173 of the housing 1170 and the vibration transmission module 200 will be given below.

Further, the body part 1172 of the housing 1170 may include a connecting part 1176 that can be coupled to the bracket 1150 described above. The connecting part 1176 may have a cylindrical shape and be screwed to the bracket 1150, but the present invention is not limited thereto.

Further, the lower surface of the housing 1170 may include a fixing groove 1177 into which a substrate, for example, a printed circuit board (PCB), may be provided.

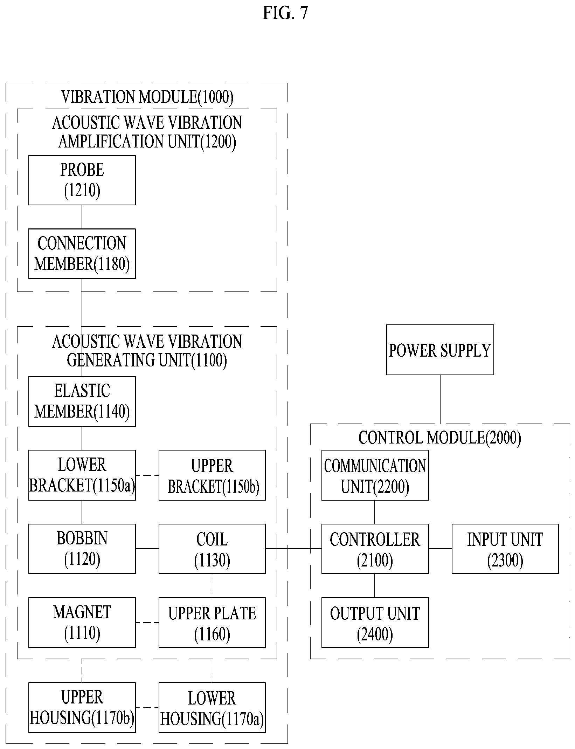

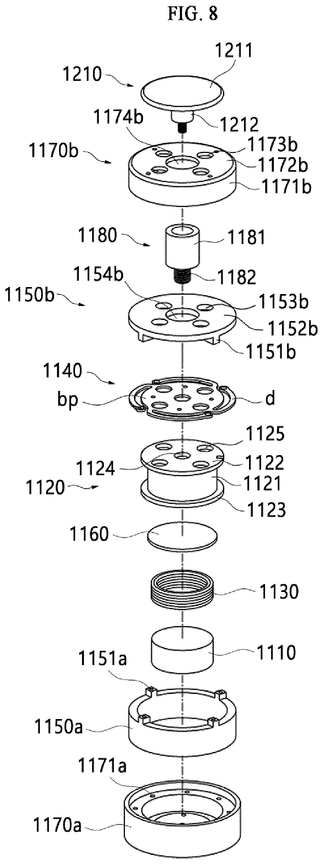

FIG. 7 is a block diagram for describing components constituting an acoustic wave vibration apparatus according to a third embodiment, and FIG. 8 is a diagram for describing components constituting a vibration module according to the third embodiment. Hereinafter, the vibration module according to the third embodiment will be described with reference to FIGS. 7 and 8.

Referring to FIGS. 7 and 8, a vibration module 1000 according to the third embodiment may include an acoustic wave vibration generating unit 1100, an acoustic wave vibration amplification unit 1200, and a housing 1170. In this case, the acoustic wave vibration generating unit 1100 of the vibration module 1000 according to the third embodiment may include a magnet 1110, an upper plate 1160, a coil 1130, a bobbin 1120, an upper bracket 1150b, a lower bracket 1150a, and an elastic member 1140, and the acoustic wave vibration amplification unit 1200 may include a probe 1210 and a connecting member. Further, the housing 1170 of the vibration module 1000 according to the third embodiment of the present invention may include an upper housing 1170b and a lower housing 1170a.

Meanwhile, the magnet 1110, the upper plate 1160, the bobbin 1120, the coil 1130, the lower bracket 1150a, the elastic member 1140, and the probe 1210 of the vibration module 1000 according to the third embodiment correspond to the magnet 1110, the upper plate 1160, the bobbin 1120, the coil 1130, the lower bracket 1150a, the elastic member 1140, and the probe 1210 of the vibration module 1000 according to the second embodiment, respectively, and contents thereof have been described above and thus detailed descriptions thereof will be omitted.

Hereinafter, the upper bracket 1150b, the connecting member, and the housing 1170, which are components of the vibration module 1000 according to the third embodiment, will be described in detail with reference to the drawings.

The vibration module 1000 according to the third embodiment of the present invention may include a plate-shaped upper bracket 1150b.

The upper bracket 1150b may have an upper surface 1152b formed in a direction opposite to the probe 1210 described above, and a lower surface that can be coupled to the elastic member 1140 and the lower bracket 1150a. The upper bracket 1150b may cover the elastic member 1140, and thus the elastic member 1140 may not be directly exposed to the outside. Accordingly, no external force is applied to the elastic member 1140, and thus the elastic member 1140 may provide an elastic force without external intervention.

The upper surface 1152b of the upper bracket 1150b may be formed to have a shape corresponding to a shape of the upper surface of the lower bracket 1150a. For example, the upper bracket 1150b may be formed to have a circular plate shape.

The upper surface 1152b of the upper bracket 1150b may have a plate shape in which at least a part is open. That is, at least a part of the upper surface 1152b of the upper bracket 1150b may have an opening, and the opening may be an insertion hole 1154b or a heat dissipation hole 1153b.

The insertion hole 1154b may be formed in the center of the upper surface 1152b of the upper bracket 1150b. A connection member 1180 which will be described below may be inserted into the insertion hole 1154b formed in the upper surface 1152b of the upper bracket 1150b to pass therethrough. Meanwhile, in order to minimize eccentricity that may occur in the vibration module 1000 when the human body is stimulated due to the vibration of the vibration module 1000, the insertion hole 1154b may be formed in the center of the upper surface 1152b of the upper bracket 1150b.

At least one heat dissipation hole 1153b may be provided in the upper surface 1152b of the upper bracket 1150b. Further, the heat dissipation hole 1153b may be provided on an outside of the insertion hole 1154b. The heat dissipation hole 1153b formed in the upper bracket 1150b may be formed to have a position, a size, and a shape corresponding to those of the heat dissipation hole formed in the bobbin 1120 and the elastic member 1140 described above. In this case, since the function, action, and effect of the heat dissipation hole 1153b formed in the upper surface 1152b of the upper bracket 1150b are the same as those of the heat dissipation hole 1125 formed in the upper surface 1122 of the bobbin 1120 described above, detailed descriptions thereof will be omitted.

Meanwhile, a protrusion 1151b that can be coupled to the elastic member 1140 and the upper surface of the lower bracket 1150a may be formed on the lower surface of the upper bracket 1150b. That is, the upper bracket 1150b may be coupled to the elastic member 1140 and the upper surface of the lower bracket 1150a, and more specifically, the protrusion 1151b formed on the lower surface of the upper bracket 1150b and a plurality of coupling protrusions 1151a formed on the upper surface of the lower bracket 1150a may be coupled to each other.

The upper bracket 1150b may be coupled to the lower bracket 1150a so that the elastic member 1140 is disposed between the lower surface of the upper bracket 1150b and the upper surface of the lower bracket 1150a. That is, in a state in which the coupling hole formed at an end of a damper d of the elastic member 1140 is located between the protrusion 1151b formed on the lower surface of the upper bracket 1150b and the plurality of coupling protrusions 1151a formed on the upper surface of the lower bracket 1150a, the upper bracket 1150b may be coupled to the elastic member 1140 and the lower bracket 1150a. In this case, the upper bracket 1150b, the elastic member 1140, and the lower bracket 1150a may be coupled in a screw coupling method, but the present invention is not limited thereto.

The protrusion 1151b formed on the lower surface of the upper bracket 1150b may be formed to have a position, number, size, or shape corresponding to those of the plurality of coupling protrusions 1151a formed on the upper surface of the lower bracket 1150a.

The vibration module 1000 according to the third embodiment of the present invention may include the connection member 1180.

The connection member 1180 may include a body part 1181 having an inner space and a connecting part 1182 for fixing the body part 1181 to an inside of the vibration module 1000.

Since the vibration module 1000 according to the third embodiment further includes the upper bracket 1150b and/or the upper housing 1170b, the connecting part 1212 of the probe 1210 should be longer than that of the first or second embodiment in order for the probe 1210 to be coupled to the elastic member 1140 and the bobbin 1120 without a separate member. Accordingly, when the vibration module 1000 vibrates, durability of the probe 1210 may be reduced. Therefore, in the vibration module 1000 according to the third embodiment, the connection member 1180 that can accommodate the connecting part 1212 of the probe 1210 may be disposed between the elastic member 1140 and the probe 1210, and thus the durability of the probe 1210 may be enhanced.

Further, the connection member 1180 may be disposed between the upper bracket 1150b and the upper housing 1170b so that the upper bracket 1150b and the upper housing 1170b may serve to fix the connection member 1180. Accordingly, even when an external force is applied to the probe 1210, the connection member 1180 may not be tilted.

Meanwhile, in FIG. 8, although one probe 1210 is illustrated, the probe 1210 may have various shapes, and thus the body part 1181 of the connection member 1180 may be formed to be compatible with the probe 1210 of various shapes so as to accommodate the probe 1210 of various shapes.

The body part 1181 of the connection member 1180 may have a cylindrical shape having an inner space. A structure in which the connecting part 1212 of the probe 1210 described above may be screwed may be formed in the inner space. Or course, the shape of the body part 1181 is not limited thereto, and the body part 1181 of the connection member 1180 may be formed to have the size and the shape corresponding to those of the insertion hole 1154b formed in the upper surface 1152b of the upper bracket 1150b described above.

Meanwhile, the connecting part 1182 of the connection member 1180 may be formed in the form of a volt that can be screwed to the elastic member 1140 and the bobbin 1120 described above, but the present invention is not limited thereto. That is, the connection member 1180 may be coupled to the elastic member 1140 and the bobbin 1120 through the connecting part 1182 of the connection member 1180. More specifically, the connection member 1180 may be coupled to the coupling hole formed in the center of the body part bp of the elastic member 1140 and to the coupling hole 1124 formed in the center of the upper surface 1122 of the bobbin 1120 through the connecting part 1182 of the connection member 1180.

In other words, the connecting part 1212 of the probe 1210 may be coupled to the body part 1181 of the connection member 1180, the body part 1181 of the connection member 1180 may be inserted and disposed into the insertion hole 1154b of the upper bracket 1150b, and then the connecting part 1182 of the connection member 1180 may be coupled to the elastic member 1140 and the bobbin 1120, and thus, when the elastic member 1140 and the bobbin 1120 generate vibration in a vertical direction when the vibration module 1000 is operated, the connection member 1180 may transmit the vertical vibration of the elastic member 1140 and the bobbin 1120 to the probe 1210.

As another example, the probe 1210 or the connection member 1180 may not be coupled to the bobbin 1120 but may be coupled only to the elastic member 1140. That is, the probe 1210 or the connecting member 1180 may be attached to the elastic member 1140.

The vibration module 1000 according to the third embodiment of the present invention may include the housing 1170.

The housing 1170 according to the third embodiment of the present invention may include the lower housing 1170a having a cylindrical shape with an open upper part, and the upper housing 1170b having a cylindrical shape with an open lower part.

The lower housing 1170a is a component corresponding to the housing 1170 of the vibration module 1000 according to the second embodiment of the present invention, and a detailed description thereof will be omitted since the above components have been described above.

The upper housing 1170b may be located on an uppermost side of the vibration module 1000 and may provide a space that can accommodate the connection member 1180, the upper bracket 1150b, the elastic member 1140, and the like, and thus may protect parts included in the upper housing 1170b. Accordingly, the upper housing 1170b may perform a function of maintaining durability of the parts accommodated in the upper housing 1170b even when an external force acts on the vibration module 1000 from the outside.

Further, the upper housing 1170b may accommodate the connection member 1180 in the inner space and, at the same time, fix the connection member 1180, and thus may prevent the probe 1210 and/or the connection member 1180 from being tilted due to the vibration of the vibration module 1000.

The upper housing 1170b may provide an inner space having a cylindrical shape with an open lower part. That is, the inner space of the upper housing 1170b may provide a space that can accommodate at least some of the lower bracket 1150a, the bobbin 1120, the elastic member 1140, the upper bracket 1150b, and the connection member 1180 described above.

An upper surface 1172b of the upper housing 1170b may be provided to have a plate shape in which at least a part thereof is open. That is, at least a part of the upper surface 1172b of the upper housing 1170b may have an opening, and the opening may be an insertion hole 1174b or a heat dissipation hole 1173b.

The insertion hole 1174b may be formed in the center of the upper surface 1172b of the upper housing 1170b. The connection member 1180 which will be described below may be inserted into the insertion hole 1174b formed in the upper surface 1172b of the upper housing 1170b to pass therethrough. Meanwhile, in order to minimize eccentricity that may occur in the vibration module 1000 when the human body is stimulated due to the vibration of the vibration module 1000, the insertion hole 1174b may be formed in the center of the upper surface 1172b of the upper housing 1170b.

At least one heat dissipation hole 1173b may be provided in the upper surface 1172b of the upper housing 1170b. Further, the heat dissipation hole 1173b may be provided in an outside of the insertion hole 1174b. The heat dissipation hole 1173b formed in the upper housing 1170b may be formed to have a position, a size, and a shape corresponding to those of the heat dissipation hole formed in the bobbin 1120 and the elastic member 1140 described above. In this case, since the function, action, and effect of the heat dissipation hole 1173b formed in the upper surface 1172b of the upper housing 1170b are the same as those of the heat dissipation hole 1125 formed in the upper surface 1122 of the bobbin 1120 described above, detailed descriptions thereof will be omitted.

Meanwhile, the upper housing 1170b may cover the open upper part of the lower housing 1170a. That is, the upper housing 1170b and the lower housing 1170a may be coupled to each other to provide a space that can accommodate parts therein.

Meanwhile, at least a part of central regions of the magnet 1110, the upper plate 1160, and the housing 1170 may be open. An open hole may be formed in the central regions of the magnet 1110, the upper plate 1160, and the housing 1170, and the open hole may provide a space through which a connection shaft can pass.

The connection shaft may be formed to have a circular tube shape, and the connection shaft may be formed to pass through the elastic member 1140, the bobbin 1120, the upper plate 1160, the magnet 1110, and the housing 1170.

The connection shaft may be connected to the elastic member 1140 and may vibrate in both directions.

The vibration apparatus comprises a connecting shaft that penetrates the elastic member, the bobbin, the magnet and the housing, and wherein, the connecting shaft further comprises the probe vibrating in a vertical direction corresponding to the bobbin at the lower part of the connecting shaft.

That is, the probes 1210 may be provided on the upper and lower sides of the acoustic wave vibration generating unit 1100. Accordingly, when the vibration module 1000 vibrates, massage may be provided to the human body in both directions.

The control module of the acoustic wave vibration apparatus 100 according to the embodiment may include a controller 2100.

The controller 2100 may control the vibration module and the output module. The controller 2100 may control an input unit 2300, a communication unit 2200, and an output unit 2400. The controller 2100 may control data to be transmitted to or received from the communication unit 2200 of the acoustic wave vibration apparatus 100 as the forms of various embodiments. That is, by the controller 2100, the data received from the user may be transmitted to the communication unit 2200 of the acoustic wave vibration apparatus 100, and the data received by the acoustic wave vibration apparatus 100 from the server may be received from the communication unit 2200.

The acoustic wave vibration apparatus 100 according to the embodiment may include the communication unit 2200.

The communication unit 2200 is a component that transmits and receives data. The communication unit 2200 may transmit and receive data from a user device, a remote controller, or a server. The communication unit 2200 may include a wireless module for wireless communication such as third-generation (3G) mobile telecommunications, fourth-generation (4G) mobile telecommunications, or Long-Term Evolution (LTE). Further, the communication unit 2200 may perform short-distance wireless communication, and a short-distance wireless communication unit 2200 may include at least one of a Bluetooth module, a radio frequency identification (RFID) module, an infrared communication module, an ultra-wideband (UWB) module, and a Zigbee module, but the present invention is not limited thereto.

The acoustic wave vibration apparatus 100 according to the embodiment may include the input unit 2300.

The input unit 2300 is a component that receives data for controlling the acoustic wave vibration apparatus from the user of the acoustic wave vibration apparatus 100. For example, the data may refer to a sound source having a specific frequency or data about the intensity of the sound source. Further, the data received from the input unit 2300 may be various pieces of other information and may refer to, for example, data about a massage mode.

The acoustic wave vibration apparatus 100 according to the embodiment may include a media output unit.

The media output unit may be a component that outputs media data to the user of the acoustic wave vibration apparatus 100 and may be referred to as a module like a display such as a liquid-crystal display (LCD). For example, the media output unit may display a specific frequency of a sound source input through the input unit 2300. As another example, the media output unit may display a degree of the intensity of the sound source input through the input unit 2300.

The acoustic wave vibration apparatus 100 according to the embodiment may include a sound source output unit.

The sound source output unit is a component that outputs a sound source having a specific frequency to the user of the acoustic wave vibration apparatus 100.

For example, the sound source output unit may be a component that outputs the sound source through the vibration module 1000. As another example, the sound source output unit may be a component that outputs a sound source through a speaker or the like.

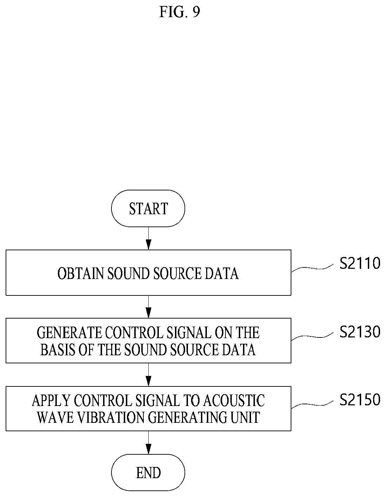

FIG. 9 is a flowchart for describing a series of operations performed by a controller 2100 according to an embodiment.

Referring to FIG. 9, the controller 2100 may perform an operation S2110 of obtaining sound source data input through the input unit 2300, an operation S2130 of generating a control signal on the basis of the sound source data, and an operation S2150 of applying the control signal to the acoustic wave vibration generating unit.

The operation S2110 of obtaining the sound source data refers to obtaining, by the controller 2100, the sound source data from an outside or inside of the acoustic wave vibration apparatus 100 and, more specifically, refers to obtaining, by the controller 2100, the sound source data from an external device (e.g., a mobile device, a sound source server, etc.) through the communication unit 2200 or loading the sound source data pre-stored in a storage unit (not illustrated) of the acoustic wave vibration apparatus 100.

The sound source data may represent information necessary to generate an acoustic wave and include frequency information of the acoustic wave and/or intensity information of the acoustic wave. Further, the sound source data may include a digital sound source in a digital format and an analog sound source in an analog format. In this case, the sound source data may be output from the output unit 2400 or output in the form of vibration from the vibration module 1000. Further, the frequency information may refer to frequency information of an audible frequency band.

The operation S2130 of generating the control signal on the basis of the sound source data may refer to adjusting a frequency and/or intensity of the sound source data obtained by the controller 2100 and then generating the control signal on the basis of the adjusted sound source data. Of course, in some embodiments, the controller 2100 may generate a control signal on the basis of the sound source data without adjusting the frequency and/or intensity of the sound source data.

The operation S2150 of applying the control signal to the acoustic wave vibration generating unit 1100 may refer to applying, by the controller 2100, the control signal to the output unit 2400 and/or the vibration module 1000 so that the control signal having a specific frequency and intensity is output from the output unit 2400 and/or the vibration module 1000. Further, the control signal applied to the output unit 2400 and the control signal applied to the vibration module 1000 may be the same as or different from each other. As a specific example, when the controller 2100 applies the control signal to the vibration module 1000, the control signal may be an electrical signal and the controller 2100 may apply the control signal to the coil 1130. In this case, the controller 2100 may adjust the intensity of the control signal to adjust the intensity of the vibration generated by the acoustic wave vibration generating unit 1100. That is, the controller 2100 may apply the control signal of which the intensity is adjusted to the acoustic wave vibration generating unit 1100.

Meanwhile, the controller 2100 may control the media output unit to output a frequency type and a degree of the intensity of the sound source data input by the user through a display or the like. Further, the controller 2100 may control the sound source data having a specific frequency and intensity input by the user to be output to a speaker or the like through the output unit. Further, the controller 2100 may control the sound source data input by the user to be output in the form of vibration through the acoustic wave vibration generating unit 1100. In other words, the controller 2100 may control one piece of sound source data to be simultaneously output from the vibration module 1000 and the output unit 2400. Further, in another embodiment, different pieces of sound source data may be simultaneously output from the vibration module 1000 and the output unit 2400.

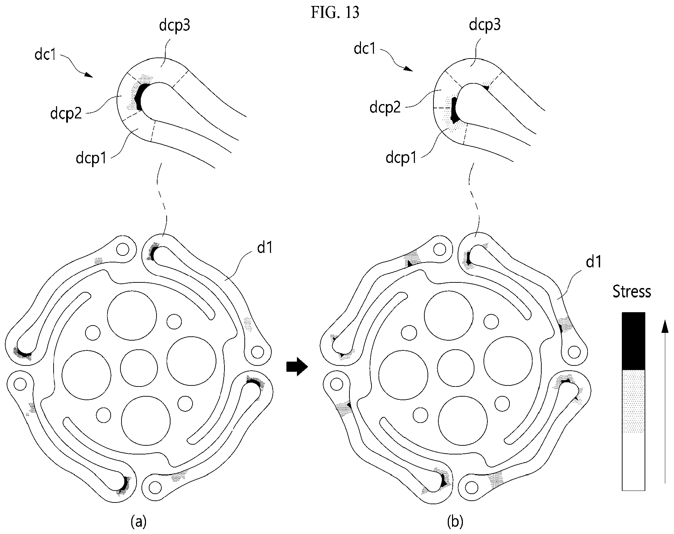

Hereinafter, an elastic member 1140 according to an embodiment of this specification will be described in more detail with reference to FIGS. 13 to 17.

The elastic member 1140 is one of the main components involved in vibration of the vibration module 1000, and the vibration efficiency, stability, or durability of the vibration module 1000 may be increased or lowered according to the size, shape, and material of the elastic member 1140. In this case, since the vibration efficiency and durability of the vibration module 1000 tend to be inversely proportional to each other, it is necessary to adjust the balance therebetween. For the above reason, a more elaborate and detailed design of the elastic member 1140 is required as will be described below.

The elastic member 1140 may refer to an object or means having an elastic force or a restoring force. For example, the elastic member 1140 may include a steel spring, a rubber spring, or the like. More specifically, the elastic member 1140 may include a coil spring that is tensioned or contracted, a plate spring including a plurality of dampers in a flat shape, a diaphragm spring, or the like.

The elastic member 1140 may include various components. For example, the elastic member 1140 may include at least one component of metals such as carbon (C), silicon (Si), beryllium (Be), chromium (Cr), phosphorus (P), sulfur (S), manganese (Mn), nickel (Ni), and the like. Physical properties of the elastic member 1140, such as weight, mass, an elastic modulus, tensile strength, yield strength, and the like, may be specified according to the components contained therein.

Hereinafter, for convenience of description, the case is mainly described in which the elastic member 1140 is formed as a flat spring, the body part of the elastic member 1140 is formed as a plate, and the elastic part of the elastic member 1140 is formed as a damper d extending from the plate, but the scope of this specification is not limited thereto, and in addition to the flat spring, other types of springs described above, and furthermore, an object having an elastic force, may similarly be applied to the elastic member 1140.

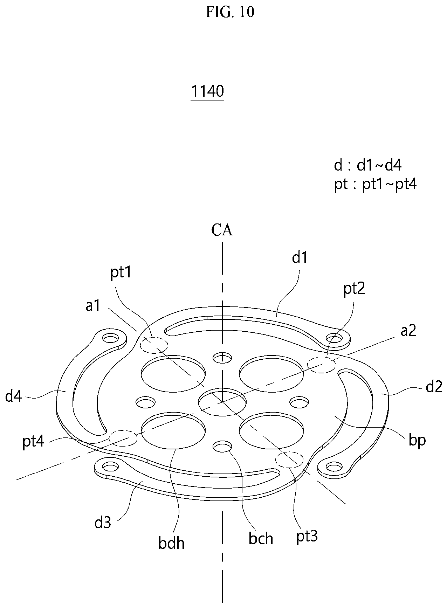

FIG. 10 is a diagram illustrating an elastic member 1140 according to an embodiment of this specification.

Referring to FIG. 10, the elastic member 1140 may include a body part bp and at least one damper d. For example, the elastic member 1140 may include a body part bp and a plurality of dampers d extending from the body part bp, and the body part bp and the dampers d may perform the following functions. The body part bp and the dampers d may provide an elastic force. For example, when the vibration module 1000 is driven, the bobbin 1120 may be moved in position and the body part bp connected to the bobbin 1120 may be moved up or down with respect to a central axis CA. In this case, the dampers d may be elastically deformed as described below to provide an elastic force in a direction opposite to or in the same direction as the moving direction of the body part bp, and the body part bp may transmit the elastic force to the bobbin 1120 connected thereto, and as a result, the bobbin 1120 may vibrate up and down.

The body part bp may be physically coupled to the vibration amplification unit 1200. For example, the vibration amplification unit 1200 may be inserted into one surface of the body part bp along the central axis CA or parallel to the central axis CA. The above-described coupling may be implemented by at least one of screw coupling, force-fitting coupling, magnetic coupling, and combinations thereof and, for the above-described coupling, the body part bp may include a central hole in the center thereof as illustrated in FIG. 10.

The body part bp may be physically coupled to the bobbin 1120. For example, the bobbin 1120 may be coupled to the other surface of the body part bp. Specifically, the bobbin 1120 and the body part bp may be coupled in at least one of screw coupling, force-fitting coupling, magnetic coupling, and combinations thereof. For the above-described coupling, as illustrated in FIG. 10, the body part bp may include a plurality of body part coupling holes bch disposed symmetrically with respect to the central axis CA. In this case, the body part bp may be in surface contact with the bobbin 1120 through the plurality of body part coupling holes bch disposed symmetrically, thereby preventing noise from being generated due to the vibration of the elastic member 1140.

Meanwhile, the body part bp may be fixed by the coupling of the bobbin 1120 and the vibration amplification unit 1200 while being disposed between the bobbin 1120 and the vibration amplification unit 1200. For example, the vibration amplification unit 1200 may be directly coupled to the bobbin 1120 or may be coupled to the bobbin 1120 through the connection member 1180 and the elastic member 1140 may be disposed therebetween, and thus the elastic member 1140, the vibration amplification unit 1200, and the bobbin 1120 may be coupled to each other by the pressing of the vibration amplification unit 1200 and the bobbin 1120.

The body part bp may include a body part heat dissipation hole bdh. For example, the body part bp may include a plurality of body part heat dissipation holes bdh disposed with respect to the central axis CA in order to discharge heat generated when the vibration module 1000 is driven to the outside. More specifically, heat generated from the coil 1130 due to internal resistance in the vibration module 1000 or heat generated by friction between the components of the vibration module 1000 may be transmitted to the elastic member 1140, and the elastic member 1140 may discharge the heat transmitted through the body part heat dissipation holes bdh.

Here, the body part coupling holes bch and the body part heat dissipation holes bdh may be disposed in the body part bp so as not to overlap each other. For example, referring again to FIG. 10, the body part coupling holes bch and the body part heat dissipation holes bdh may be alternately disposed in the body part bp in a clockwise or counterclockwise direction.

In addition, here, as many of the body part coupling holes bch and the body part heat dissipation holes bdh as is appropriate may be disposed in the body part bp. For example, in consideration of the above-described physical properties of the elastic member 1140, the body part bp may include four body part coupling holes bch and four body part heat dissipation holes bdh.

The damper d may connect the body part bp to the bracket 1150 or the housing 1170. For example, an end of the damper d extending from the body part bp may be connected to the bracket 1150 or the housing 1170. In this case, the end of the damper d may be fixed to the bracket 1150 or the housing 1170 so that the damper d may be elastically deformed according to the movement of the coil 1130 and the bobbin 1120 due to an electromagnetic force, and thus the elastic member 1140 coupled to the bobbin 1120 may vibrate around the end of the fixed damper d.

The dampers d may be implemented so that an appropriate number may withstand a load generated when the elastic member 1140 vibrates. For example, referring again to FIG. 10, the elastic member 1140 may include first to fourth dampers d1, d2, d3, and d4.

The dampers d may extend from the body part bp. For example, as illustrated in FIG. 10, the first damper d1 may extend from a first point pt1 of the body part bp, the second damper d2 may extend from a second point pt2 of the body part bp, the third damper d3 may extend from a third point pt3 of the body part bp, and the fourth damper d4 may extend from a fourth point pt4 of the body part bp.

Here, a point pt at which the damper d extends from the body part bp may be set symmetrically with respect to the central axis CA of the elastic member 1140. For example, as illustrated in FIG. 10, when the elastic member 1140 includes four dampers d, first to fourth points pt1, pt2, pt3, and pt4 may be set to be positioned on a virtual first axis a1 and a virtual second axis a2 that divide the elastic member 1140 into four.

The dampers d may extend to face an outer peripheral surface of the body part bp or may extend along the outer peripheral surface. For example, as illustrated in FIG. 10, the first to fourth dampers d1, d2, d3, and d4 may extend along the outer peripheral surface of the body part bp in a clockwise or counterclockwise direction. As another example, the first to fourth dampers d1, d2, d3, and d4 may extend from the body part bp in a direction away from the central axis CA while drawing a parabolic line.

Here, the dampers d may extend along the outer peripheral surface of the body part bp, and the different dampers d may not be in physical contact with each other. For example, as illustrated in FIG. 10, when the first damper d1 and the second damper d2 extend from the first point pt1 and the second point pt2 positioned on the virtual first axis a1 and the virtual second axis a2 that divide the body part bp into four, the first damper d1 and the second damper d2 may not be in contact with each other. Specifically, the first damper d1 may extend from the first point pt1 and extend to have a length so that an end of the first damper d1 does not cross the second axis a2, and the second damper d2 may extend from the second point pt2 and extend to have a length so that an end of the second damper d2 does not cross the first axis a1. Further, the above-described contents may be applied to the third damper d3 and the fourth damper d4 in the same manner.

The damper d may include a damper coupling hole. For example, as illustrated in FIG. 10, each of the first to fourth dampers d1, d2, d3, and d4 may include a damper coupling hole in the end thereof, and the elastic member 1140 may be physically coupled to the bracket 1150 or the housing 1170 through the damper coupling holes. Here, at least one of force-fitting coupling, screw coupling, magnetic coupling, compression coupling, close coupling, and combinations thereof may be used as the above-described coupling.

In the above, for convenience of description, the case is mainly described in which the elastic member 1140 includes four dampers d, but the scope of this specification is not limited thereto. For example, the elastic member 1140 may include two dampers d which are disposed or extend with respect to a virtual axis that divides the body part bp into two, may include three dampers d which are disposed or extend with respect to three virtual axes that divide the body part bp into three, or may include five dampers d which are disposed or extend with respect to five virtual axes that divide the body part bp into five. Meanwhile, based on the length of the damper d extending from the body part bp and the width of the damper d, an elastic modulus indicating a degree of elasticity or restoration of the elastic member 1140 or a safety coefficient indicating a degree of not being damaged by driving of the vibration module 1000 or external pressure may be determined. For example, as the length of the damper d extending from the body part bp is increased, the elastic force of the elastic member 1140 may be increased so that the elastic modulus may be increased. As another example, as the length of the damper d extending from the body part bp is increased, the elastic member 1140 may vibrate due to the driving of the vibration module 1000 and the damper d may be easily damaged so that the safety coefficient may be lowered.

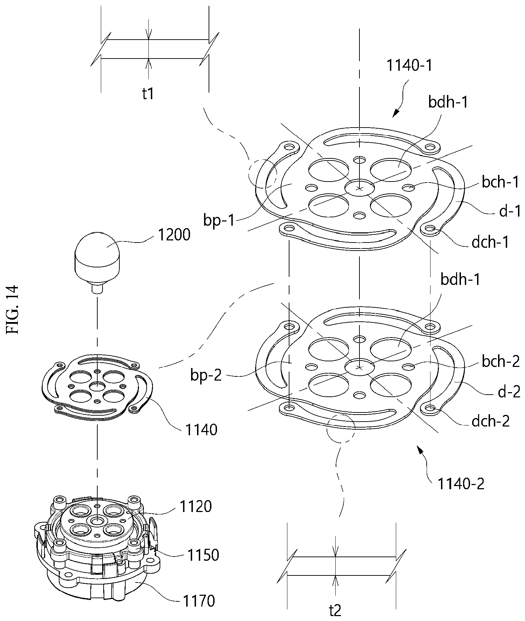

Hereinafter, an elastic member 1140 including a damper d extending from a body part bp in a specific shape so as to increase a safety coefficient while increasing the elastic modulus will be described with reference to FIGS. 14 to 16.

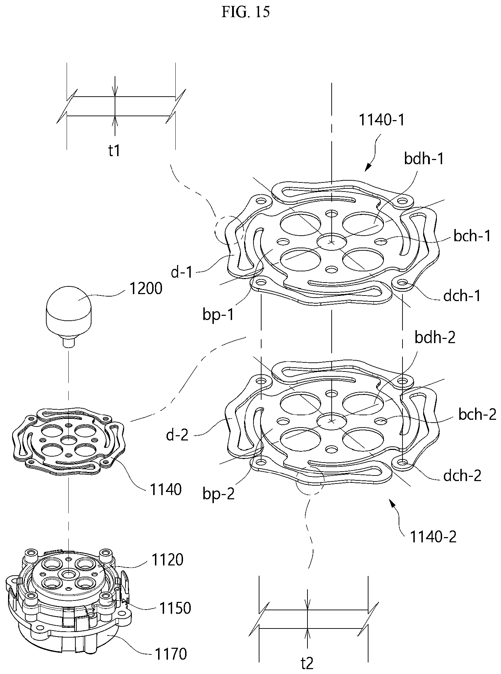

FIGS. 14 and 15 are diagrams illustrating an elastic member 1140 including dampers d having parts that are bent in different directions for each part according to an embodiment of this specification. Hereinafter, the contents described in FIG. 10 may be applied in the same manner unless otherwise specified.

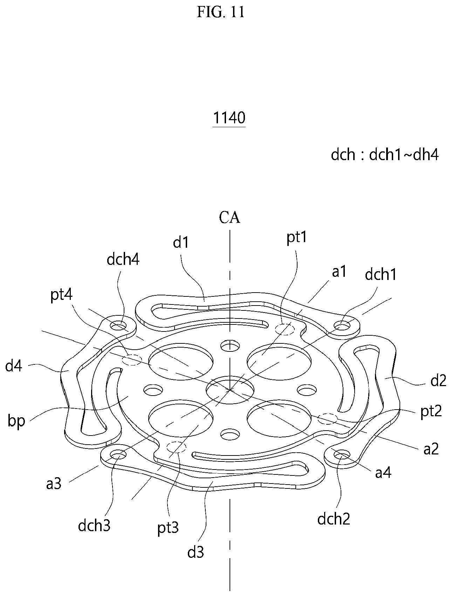

Referring to FIG. 11, the dampers d may extend from the body part bp while changing a bending direction for each part. Alternatively, the dampers d may extend from the body part bp, but the extending direction thereof may be changed during the extension. Specifically, the dampers d may extend from the body part bp in a clockwise or counterclockwise direction and may extend in a direction away from the central axis CA and then extend in a counterclockwise or clockwise direction. A shape of the damper d will be described below in detail.

The damper d may extend from a specific point of the body part bp. For example, referring again to FIG. 11, first to fourth dampers d1, d2, d3, and d4 may extend from first to fourth points pt1, pt2, pt3, and pt4 of the body part bp. Here, a point pt at which the damper d extends from the body part bp is the same as described in FIG. 10 above. For example, the first to fourth points pt1, pt2, pt3, and pt4 at which the first to fourth dampers d1, d2, d3, and d4 extend from the body part bp may be disposed on a first axis a1 and a second axis a2 that divide the body part bp into four.

The damper d may include a damper coupling hole dch. For example, each of the first to fourth dampers d1, d2, d3, and d4 may include first to fourth damper coupling holes dch1, dch2, dch3, and dch4 in an end thereof. The damper d may be coupled to the bracket 1150 or the housing 1170 through the damper coupling hole dch.

The damper coupling hole dch may be disposed at a specific position based on the body part bp. For example, as illustrated in FIG. 11, the first to fourth damper coupling holes dch1, dch2, dch3, and dch4 may be disposed symmetrically with respect to the central axis CA of the body part bp. Specifically, the first to fourth damper coupling holes dch1, dch2, dch3, and dch4 may be disposed on a third axis a3 and a fourth axis a4 that divide the body part bp into four. Here, the first damper coupling hole dch1 and the third damper coupling hole dch3 may be disposed on the third axis a3, and the second damper coupling hole dch2 and the fourth damper coupling hole dch4 may be disposed on the fourth axis a4. In this case, the length of the damper d may be set according to the position of the damper coupling hole dch, as described below.

There is a specific positional relationship between the point pt at which the damper d extends from the body part bp and the damper coupling hole dch.

For example, the point pt at which the damper d extends from the body part bp and the damper coupling hole dch may have a preset angle with respect to the central axis CA of the body part bp. Specifically, the first to fourth damper coupling holes dch1, dch2, dch3, and dch4 may be disposed on a virtual extension line rotated by a preset angle in a clockwise or counterclockwise direction with respect to the central axis CA from a virtual extension line connecting the center of the body part bp to the first to fourth points pt1, pt2, pt3, and pt4. For example, the first axis a1 on which the first and third points pt1 and pt3 are disposed and the third axis a3 on which the first and third damper coupling holes dch1 and dch3 are disposed may meet at a central point of the body part bp or may have a predetermined angle with respect to the central axis CA, and the second axis a2 on which the second and fourth points pt2 and pt4 are disposed and the fourth axis a4 on which the second and fourth damper coupling holes dch2 and dch4 are disposed may meet at the central point of the body part bp or may have a predetermined angle with respect to the central axis CA.

As described above, when the point pt at which the damper d extends from the body part bp and the damper coupling hole dch are not positioned on the same line with respect to the center of the body part bp, the damper coupling hole dch may be disposed in a space between a point pt at which one damper d extends and another damper d. In this case, the overall size of the elastic member 1140, particularly, the farthest distance from the central axis CA, of the elastic member 1140, may be reduced, and as a result, the volume of the vibration module 1000 may be reduced or maintained.

Meanwhile, the point pt at which the damper d extends from the body part bp and the damper coupling hole dch may be disposed on the same extension line based on the center of the body part bp. For example, the first and third damper coupling holes dch1 and dch3 may be located on the first axis a1 on which the first and third points pt1 and pt3 described above are located, and the second and fourth damper coupling holes dch2 and dch4 may be located on the second axis a2 on which the second and fourth points pt2 and pt4 are located.

The length of the damper d may be determined according to an arrangement position of the damper coupling hole dch and the point pt at which the damper d extends from the body part bp. For example, the length of the damper d may be determined as a length from the point pt of the body part bp to the damper coupling hole dch along the damper d. Specifically, as illustrated in FIG. 11, the first damper d1 may extend by a first length from the first point pt1 in a counterclockwise direction with respect to the central axis CA and extend by a second length to the position at which the first damper coupling hole dch1 is disposed in a clockwise direction. In this case, the arrangement position of the first damper coupling hole dch1 may be spaced a predetermined angle from the first point pt1 in a clockwise direction with respect to the central axis CA so that the second length may be set to be greater than the first length. Meanwhile, the elasticity efficiency of the elastic member 1140 may be improved as the length of the damper d is increased and it is easier to increase the length of the outer part than that of the inner part of the damper d, as described below, and thus, due to the above-described arrangement of the damper coupling hole dch, the elasticity efficiency of the elastic member 1140 may be improved.

In the above, the damper d is described as including the damper coupling hole dch and the position at which the damper coupling hole dch is disposed, but the scope of this specification is not limited thereto, and the damper d may not include the damper coupling hole dch, and in this case, the arrangement position of the damper coupling hole dch may be understood as an arrangement position of the end of the damper d in the above description.

As described above, in the case in which the extending direction is changed while the damper d extends from the body part bp, the length at which the damper d can extend while different dampers d are not in contact with each other may be increased as compared to the case the extending direction is not changed. As a result, the elastic modulus of the elastic member 1140 may be increased and thus the vibration efficiency of the vibration module 1000 may be improved.

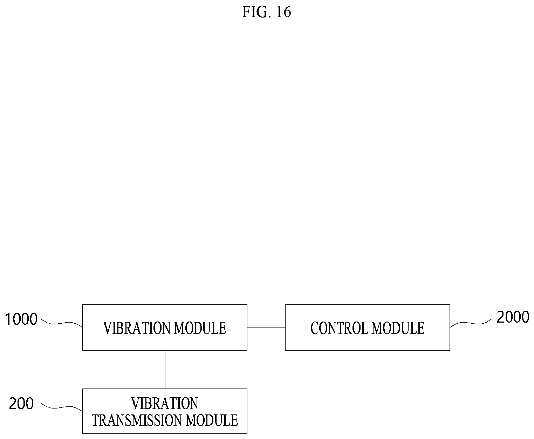

Hereinafter, the shape of the damper d for preventing a risk of damage occurring as the length of the damper d is increased in the elastic member 1140 will be described in detail with reference to FIGS. 15 and 16.

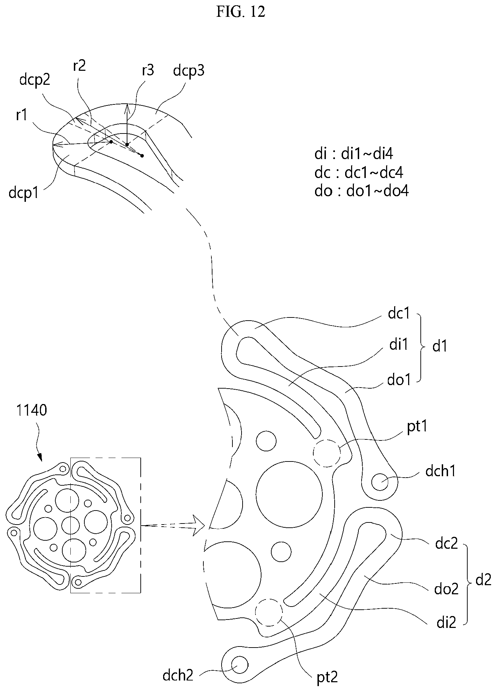

Referring to FIG. 12, in order to improve the vibration efficiency of the vibration module 1000, the damper d may include an inner part di, an outer part do, and a connection part dc. Specifically, the damper d may include the inner part di extending from the body part bp, the outer part do disposed further away from the center of the body part bp than the inner part di, and the connection part dc connecting the inner part di to the outer part do. For example, referring to FIG. 12, the first damper d1 may be composed of or divided into a first inner part di1, a first connection part dc1, and a first outer part do1, and the second damper d2 may be composed of or divided into a second inner part di2, a second connection part dc2, and a second outer part do2.

Since the damper d extends from the body part bp, a direction of bending at the inner part di and a direction of bending at the connection part dc may be different. Here, the connection part dc of the damper d extends by 90° or more from the center of the connection part dc in a bending direction different from the bending direction of the inner part di, and the length of the damper d in a limited region may be increased.

The inner part di may refer to a part of the damper d close to the center of the body part bp. For example, as illustrated in FIG. 12, the first inner part di1 may refer to a part of the first damper d1 extending to face the outer peripheral surface of the body part bp with a preset radius of curvature at the first point pt1 of the body part bp.

The outer part do may refer to a part of the damper d that is spaced further away from the body part bp than the inner part di. For example, as illustrated in FIG. 12, the first outer part do1 may refer to a part of the first damper d1 extending to face the outer peripheral surface of the first inner part di1 with a preset radius of curvature from the first inner part di1 or the first connection part dc1.

The inner part di and the outer part do may extend to different lengths. For example, the outer part do may extend to be longer than the inner part di. Specifically, as illustrated in FIGS. 14 and 15, the first point pt1 at which the first damper d1 may be connected to the body part bp and the second connection part dc2 of the second damper d2 may be spaced apart from each other to have a preset angle with respect to the central axis CA of the body part bp and the first damper coupling hole dch1 of the first damper d1 or the end of the first outer part do1 may be located in a region between the first point pt1 and the second connection part dc2, and thus the first outer part dot may extend to be longer than the first inner part di1 by an angle between the first point pt1 and the second connection part dc2 or a length corresponding to a region between the first point pt1 and the second connection part dc2. In this case, a distance from the first point pt1 to the first damper coupling hole dch1 may be smaller than a distance from the first point pt1 to the first connection part dc1 of the first damper d1.

The connection part dc may refer to a part of the damper d connecting the inner part di to the outer part do. For example, as illustrated in FIG. 12, the first connection part dc1 may refer to a part of the first damper d1 connecting the first inner part di1 to the first outer part do1.

The connection part dc may be implemented in various shapes. For example, the connection part dc may be implemented in a shape that can connect the inner part di to the outer part do, such as a U shape, a V shape, an I shape, or the like. Here, the connection part dc may change the extending direction of the damper d extending from the body part bp.

Meanwhile, since the damper d includes the inner part di, the connection part dc, and the outer part do, damage due to friction may occur between the damper d and the housing 1170 or between the damper d and the bracket 1150 when the acoustic wave vibration apparatus 100 vibrates according to the size or shape of the coupling protrusion of the housing 1170 or the bracket 1150 connected to the elastic member 1140 when the elastic member 1140 is connected to the housing 1170 or the bracket 1150. In order to prevent the damage due to the friction, a radius of the coupling protrusion should be set within a preset distance or less from the center of the damper coupling hole dch. Specifically, when the first damper d1 of the elastic member 1140 is connected to the housing 1170 or the bracket 1150 through the first damper coupling hole dch1 and the housing 1170 or the first coupling protrusion of the bracket 1150, a length from the center to the end of the first coupling protrusion may be set to be less than a distance from the center of the first damper coupling hole dch1 to the second connection part dc2 of the second damper d2 adjacent to the first damper d1.

The direction of partial bending of the damper d may be changed. Alternatively, the damper d may have a partially winding shape or a zigzag shape. Alternatively, the damper d may include convex parts and concave parts which are alternately formed in a central direction of the body part bp or in a direction perpendicular to the central axis CA. Alternatively, the damper d may include a plurality of bending points. Alternatively, the damper d may have a partially different radius of curvature. Alternatively, in the damper d, specifically, at least some of the inner part di, the connection part dc, and the outer part do of the damper d may be partially bent in different directions.

For example, in the inner part di, the direction in which the inner part di is bent while extending from the body part bp to the connection part dc may be changed. Specifically, an end of the inner part di extending from the body part bp and the other end of the inner part di connected to the connection part dc may be bent in different directions. Alternatively, the radius of curvature of the inner part di may be changed while the inner part di extends from the body part bp to the connection part dc.

As another example, the bending direction of the outer part do may be changed while the outer part do extends from the connection part dc to the end of the outer part do. Specifically, as illustrated in FIG. 12, the outer part do may extend from the connection part dc to extend to be convex in the direction of the body part bp and then extend to be concave in the direction of the body part bp. Alternatively, the radius of curvature of the outer part do may be changed while extending from the connection part dc to the end of the outer part do.

As described above, the inner part di and the outer part do may be formed so that the bending direction or the radius of curvature thereof are partially changed. In this case, the length of the damper d may be increased compared to the case in which the bending direction or the radius of curvature is constant, and thus the elastic modulus or elasticity efficiency of the elastic member 1140 may be improved. However, as the number of times the bending direction of the inner part di and the outer part do is changed increases, a manufacturing process becomes complicated and consumption cost increases, and thus only one part of the inner part di and the outer part do may be formed so that the bending direction is changed or the number of times that the bending direction is changed within each part of the inner part di and the outer part do may be limited to a preset value or less.

Meanwhile, in the above, the bending direction of the damper d of the elastic member 1140 has been described centering on a plane including one surface of the elastic member 1140, but the scope of this specification is not limited thereto. For example, unlike that illustrated in FIGS. 14 or 15, the damper d may be bent in an upward or downward direction of the elastic member 1140 or in a direction parallel to the central axis CA. Specifically, at least a part of the damper d may be bent in a first direction parallel to the central axis CA and then bent in a second direction parallel to the central axis CA and different from the first direction.

As described above, when the bending direction is changed at the inner part di and the outer part do, the stress may be distributed as described below so that durability may be improved.

As still another example, the connection part dc may include parts extending with different radii of curvature. For example, as illustrated in FIG. 12, the first connection part dc1 of the first damper d1 may include first to third parts dcp1, dcp2, and dcp3 having different radii of curvature. More specifically, the first connection part dc1 may include the first part dcp1 which extends from the first inner part di1 and has a first radius r1 of curvature, the second part dcp2 which extends from the first part dcp 1 and has a second radius r2 of curvature, and the third part dcp3 which extends from the second part dcp2, has a third radius r3 of curvature, and is connected to the first outer part do1.

Here, the first radius r1 of curvature and the third radius r3 of curvature may be smaller than or equal to the second radius r2 of curvature. In this case, the first radius r1 of curvature may be greater than or smaller than the third radius r3 of curvature.

In addition, here, the second part dcp2 may have a rod shape and the second radius r2 of curvature may be infinite. Alternatively, the second part dcp2 of the first connection part dc1 of the first damper d1 may be omitted, or the first damper d1 may further include a part other than the first to third parts dcp1, dcp2, and dcp3.

As described above, when the damper d of the elastic member 1140 extends in different bending directions for each part or includes parts having different radii of curvature, a load applied to the elastic member 1140 or a stress corresponding thereto may be distributed.

FIG. 13 illustrates diagrams illustrating stress distribution according to structural analysis of an elastic member 1140.

A stress may refer to a resistance force generated in the elastic member 1140 in response to a magnitude thereof when a load (external force) such as compression, tension, bending, and torsion is applied to the elastic member 1140 due to the operation of the acoustic wave vibration apparatus 100. Here, von Mises stress or effective or equivalent stress is used as a structural analysis method of the elastic member 1140, but other structural analysis methods may be used. As a result, a part with high stress may have a higher probability of being damaged or deformed than a part with relatively low stress.

The stress may include an allowable stress allowed when the elastic member 1140 drives the acoustic wave vibration apparatus 100 and a fracture stress that destroys. When an external force applied to the elastic member 1140 or a generated stress should be less than the allowable stress, the acoustic wave vibration apparatus 100 may be operated stably. When the damper d of the elastic member 1140 extends in different bending directions for each part as described above or includes parts having different radii of curvature, the stress may be distributed as described below.

In the elastic member 1140, the stress may be generated relatively higher in the damper d than in the body part bp. For example, as illustrated in FIG. 13, a relatively high stress may be generated in the connection part dc of the damper d of the elastic member 1140, and thus a risk of damage to the connection part dc due to the driving of the acoustic wave vibration apparatus 100 may be increased.

When the radius of curvature is partially changed in the damper d, the stress may be distributed. For example, referring again to FIG. 13, when the radius of curvature is constant in the first to third parts dcp1, dcp2, and dcp3 of the damper d as illustrated in FIG. 13A, the stress may be concentrated, and when the damper d includes the first to third parts dcp1, dcp2, and dcp3 having different radii of curvature as illustrated in FIG. 13B, the stress may be distributed. Specifically, the stress mainly generated in the second part dcp2 of the damper d may be distributed to the first and third parts dcp1 and dcp3.

In the above, the first damper d1 has been mainly described as a representative damper among the damper d of the elastic member 1140, and the same content may be applied to the second to fourth dampers d2, d3, and d4.

Further, in the above, the case in which the damper d is composed of the inner part di, the connection part dc, and the outer part do has been mainly described, but the scope of this specification is not limited thereto. For example, at least a part of the inner part di and at least a part of the connection part dc may overlap, and at least a part of the connection part dc and at least a part of the outer part do may overlap. As another example, the damper d may further include other parts in addition to the inner part di, the connection part dc, and the outer part do described above.

As described above, when the stress generated by the elastic member 1140 is distributed, a safety coefficient representing a ratio of the fracture stress and the allowable stress may be increased and accordingly, the damage and deformation of the elastic member 1140 due to the driving of the acoustic wave vibration apparatus 100 may be prevented, and thus the durability of the elastic member 1140 may be improved.

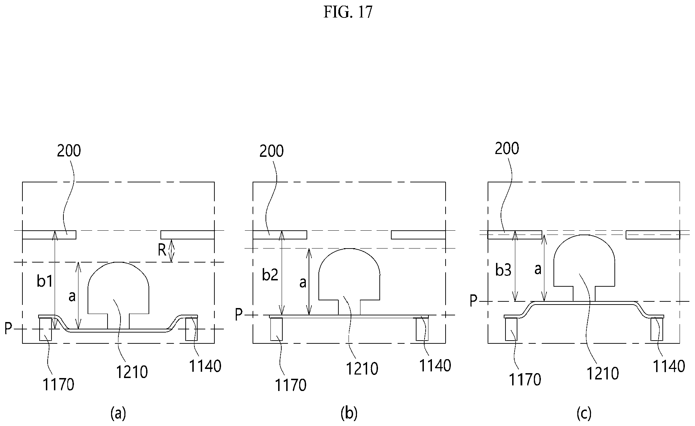

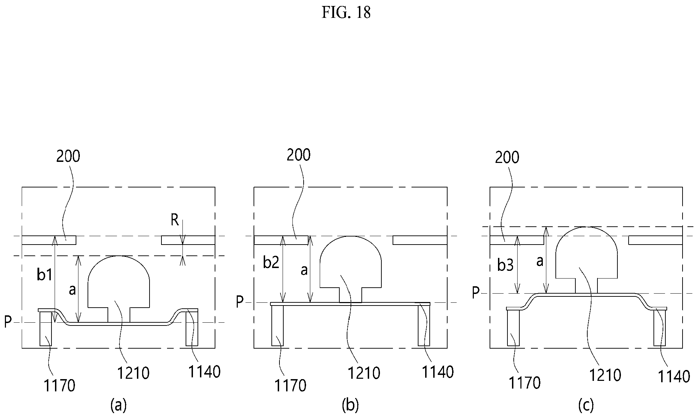

Hereinafter, a shape of another elastic member 1140 with improved stability will be described with reference to FIGS. 17 and 18.

FIGS. 17 and 18 are diagrams illustrating a vibration generating unit 1100 including a plurality of elastic members 1140 according to an embodiment of this specification.

Referring to FIG. 14, the vibration generating unit 1100 may include the plurality of elastic members 1140. For example, the vibration generating unit 1100 may include a first elastic member 1140-1 and a second elastic member 1140-2.

Here, the description of the elastic member 1140 described elsewhere in this specification may be applied to the first and second elastic members 1140-1 and 1140-2 as it is.

The first elastic member 1140-1 and the second elastic member 1140-2 may have the same shape. Alternatively, the first elastic member 1140-1 and the second elastic member 1140-2 may have partially the same shape. For example, as illustrated in FIG. 14, the first and second elastic members 1140-1 and 1140-2 may include the same body part bp and damper d. As another example, the first and second elastic members 1140-1 and 1140-2 may include the same damper d but include body parts bp having different shapes. As still another example, the first and second elastic members 1140-1 and 1140-2 may have different sizes or thicknesses, as described below.

The first elastic member 1140-1 and the second elastic member 1140-2 may be coupled to be in surface contact with each other. For example, as illustrated in FIG. 14, the first elastic member 1140-1 and the second elastic member 1140-2 may be coupled to the bobbin 1120 through the vibration amplification unit 1200 while being in surface contact with each other or overlapping each other. Alternatively, the first elastic member 1140-1 and the second elastic member 1140-2 may be coupled to the housing 1170 or the bracket 1150 through a coupling member such as a screw or a pin while being in surface contact with each other or overlapping each other.

For the above-described coupling, the first elastic member 1140-1 and the second elastic member 1140-2 may include at least one of a body part coupling hole bch, a damper coupling hole dch, and a body part heat dissipation hole bdh at positions corresponding to each other. For example, as illustrated in FIG. 14, the second elastic member 1140-2 may include a second body part coupling hole bch-2 disposed on the same vertical line as a first body part coupling hole bch-1 of the first elastic member 1140-1. Further, the second elastic member 1140-2 may include a second damper coupling hole dch-2 disposed on the same vertical line as a first damper coupling hole dch-1 of the first elastic member 1140-1.

Meanwhile, for smooth heat dissipation, the first and second elastic members 1140-1 and 1140-2 may include body part heat dissipation holes bdh at positions corresponding to each other. For example, the second elastic member 1140-2 may include a second body part heat dissipation hole bdh-2 disposed on the same vertical line as a first body part heat dissipation hole bdh-1 of the first elastic member 1140-1.

A sum of thicknesses of the first elastic member 1140-1 and the second elastic member 1140-2 may be set within a specific range. For example, a sum of a first thickness t1 of the first elastic member 1140-1 and a second thickness t2 of the second elastic member 1140-2 may be included in a range of 0.5 mm to 3.0 mm. Since the sum of the first thickness t1 and the second thickness t2 is included within the above-described range, the first elastic member 1140-1 and the second elastic member 1140-2 may have high elastic efficiency or improved durability compared to one elastic member 1140 having the same thickness.

The first elastic member 1140-1 and the second elastic member 1140-2 may have the same thickness or different thicknesses. For example, when the first elastic member 1140-1 is located on an upper end of the second elastic member 1140-2, the first thickness t1 of the first elastic member 1140-1 may be greater than or equal to the second thickness t2 of the second elastic member 1140-2. As another example, when the first elastic member 1140-1 is located on the upper end of the second elastic member 1140-2, the first thickness t1 of the first elastic member 1140-1 may be smaller than or equal to the second thickness t2 of the second elastic member 1140-2. Each of the first thickness t1 and the second thickness t2 may be set in a range of 0.1 mm to 2.5 mm. The sum of the first thickness t1 and the second thickness t2 and each of the first thickness t1 and the second thickness t2 are included in the above-described ranges, and thus the elastic member 1140 may be designed to have a more stable and high vibration amplitude within a frequency range (e.g., in a range of 80 Hz to 200 Hz) used in the acoustic wave vibration apparatus 100.

The shapes of the first elastic member 1140-1 and the second elastic member 1140-2 may be variously set. For example, as illustrated in FIG. 15, the first elastic member 1140-1 and the second elastic member 1140-2 may be implemented to have the shape of the elastic member 1140 described in FIG. 11.

The first elastic member 1140-1 and the second elastic member 1140-2 may be coupled with different bonding strengths for each part. For example, as illustrated in FIG. 14, the first and second elastic members 1140-1 and 1140-2 may be coupled so that the bonding strength between a first elastic member body part bp-1 and a second elastic member body part bp-2 is greater than the bonding strength between a first elastic member damper d-1 and a second elastic member damper d-2. Specifically, the first elastic member body part bp-1 and the second elastic member body part bp-2 may include bonding points greater than the number of bonding points between the first elastic member damper d-1 and the second elastic member damper d-2.

The first elastic member body part bp-1 and the second elastic member body part bp-2 are parts that vibrate as the bobbin 1120 is moved in position, and when the first elastic member body part bp-1 and the second elastic member body part bp-2 are not sufficiently coupled, noise may occur due to vibration. Accordingly, the first elastic member body part bp-1 and the second elastic member body part bp-2 may be coupled through at least two body part coupling holes bch symmetrically disposed with respect to the central axis CA in each body part bp in addition to the coupling by the vibration amplification unit 1200.

The first elastic member damper d-1 and the second elastic member damper d-2 are parts that determine the amplitude as the bobbin 1120 is moved in position, and elastic deformation may occur therein. When the first elastic member damper d-1 and the second elastic member damper d-2 are brought into strong contact with each other and are coupled to each other during such elastic deformation, damage may occur therebetween.

Further, when the bobbin 1120 is moved in position, a part of the first elastic member damper d-1 and a part of the second elastic member damper d-2 may be spaced a predetermined distance from each other. As described above, when two elastic members are coupled, the part of the first elastic member damper d-1 and the part of the second elastic member damper d-2 may be spaced the predetermined distance from each other, and thus stresses in the first elastic member damper d-1 and the second elastic member damper d-2 may be more efficiently distributed than those when one elastic member having the same thickness is used.

Therefore, the first elastic member damper d-1 and the second elastic member damper d-2 may be coupled only through one fixed end (e.g., the damper coupling hole dch).

As described above, when the plurality of elastic members 1140 are provided, the plurality of elastic members 1140 may be coupled through the appropriate coupling points for each part, and thus damage may be prevented and the plurality of elastic members 1140 may vibrate more stably.

In the above, when the plurality of elastic members 1140 are provided, the case in which the first elastic member 1140-1 and the second elastic member 1140-2 are included has been mainly described, but the scope of the present invention is not limited thereto, and three or more elastic members 1140 may be provided and in this case, the above-described contents may be similarly applied.

Hereinafter, various embodiments including the vibration transmission module 200 that efficiently transmits the vibration generated according to the above-described vibration module 1000 to various mechanisms or devices in which the vibration module 1000 is mounted will be described.

An embodiment of this specification may be a vibration apparatus 10 including a vibration module 1000 and a vibration transmission module 200, and another embodiment may be an application including a vibration module 1000, a vibration transmission module 200, and a vibration receiving unit S. In this case, the application may include a massage device or an exercise apparatus. In addition, various embodiments may be applied to the present invention.

Meanwhile, since contents of the vibration apparatus 10 according to an embodiment, and the vibration module 1000 and the control module 2000 included in the application according to another embodiment correspond to the contents of the vibration module 1000 and the control module 2000 described with reference to FIG. 1, the same reference numerals may be assigned to common parts and detailed descriptions thereof will be omitted.

FIG. 16 is a diagram for describing components constituting a vibration apparatus according to an embodiment.

Referring to FIG. 16, the vibration apparatus according to the embodiment may include a vibration module 1000, a control module 2000, and a vibration transmission module 200. Hereinafter the vibration transmission module 200 will be described in detail with reference to the drawings.

The vibration transmission module 200 may function to efficiently transmit vibration generated by the vibration module 1000 to a mechanism or device in which the vibration module 1000 is mounted by being coupled to at least a part of the vibration module 1000.

The vibration generated by the operation of the vibration module 1000 is transmitted to the human body through the probe 1210. In this case, when it is assumed that a range of the human body that can receive the vibration transmitted through the probe 1210 is defined as a vibration transmission range, the vibration apparatus 10 according to the embodiment may further extend the vibration transmission range by including the vibration transmission module 200.

More specifically, when power is applied to the vibration module 1000, the vibration module 1000 may vibrate according to a force induced due to an interaction between the current flowing through the coil 1130 and the magnetic field generated by the magnet 1110. In this case, the vibration generated by the vibration module 1000 is transmitted to the vibration transmission module 200. Accordingly, by vibrating not only the vibration module 1000 but also the vibration transmission module 200 together, the vibration apparatus 10 including the vibration transmission module 200 may have a wider vibration transmission range compared to the vibration module 1000 that does not include the vibration transmission module 200.

For example, when it is assumed that the vibration transmission range of the vibration module 1000 is a region in contact with the probe 1210 and the vibration is transmitted to the periphery, that is, a region around the probe 1210, the vibration apparatus 10 including the vibration transmission module 200 may have a vibration transmission range being extended to not only a peripheral region of the probe 1210 but also a peripheral region of the vibration transmission module 200 surrounding at least a part of the probe 1210.

Meanwhile, the vibration transmission module 200 may be directly or indirectly coupled to at least a part of the housing 1170 of the vibration module 1000 and may be formed to surround at least a part of the probe 1210 of the vibration module 1000 to induce dispersion of an external force with respect to the probe 1210.

As a more specific example, when the vibration module 1000 vibrates, the probe 1210 may perform a massage function while being brought into contact with the human body, and as the probe 1210 is brought into contact with the human body, the external force acts on the probe 1210. In this case, when the external force applied to the probe 1210 exceeds a predetermined value, the vibration transmission range of the probe 1210 is limited and thus vibration transmission efficiency is lowered.

The vibration transmission module 200 may be formed to surround at least a part of the probe 1210 to induce dispersion of the external force with respect to the probe 1210, and thus vibration and massage efficiency may be increased.

Hereinafter, a shape, height, and type of the vibration transmission module 200 and a method of coupling with the housing 1170 will be described in more detail.

The vibration transmission module 200 may be formed to provide an inner space. More specifically, the vibration transmission module 200 may be formed to provide the inner space that can accommodate at least a part of the vibration module 1000 described above. For example, the vibration transmission module 200 may have a rectangular parallelepiped shape or a cylindrical shape having an inner space and may be formed in various known shapes having an inner space.

Meanwhile, the vibration transmission module 200 may be provided to have an open shape in which at least a part of the upper part is open. When the vibration transmission module 200 is provided to have an open shape, the probe 1210 may be disposed to protrude into the open part, but the present invention is not limited thereto. For example, the probe 1210 may be disposed below the open part so as not to protrude to the outside of the open part. Further, when the vibration transmission module 200 has an open type, the open part may be formed to correspond to the position and shape of the probe 1210. For example, the open shape may be a circular shape.

Meanwhile, according to the open shape of the vibration transmission module 200, the vibration transmission range may include all of the peripheral region of the probe 1210 and the peripheral region of the housing 1170 and, accordingly, the vibration transmission module 200 may transmit vibrations having a variety of stronger intensities.

Meanwhile, the vibration transmission module 200 may be provided to have a closed shape in which the upper part is closed. When the vibration transmission module 200 is provided to have a closed shape, the probe 1210 may be disposed below the upper part of the vibration transmission module 200 but may be disposed not to protrude to the outside of the vibration transmission module 200. That is, when the vibration transmission module 200 is provided to have a closed shape, at least a part of the probe 1210 may be accommodated in the inner space of the vibration transmission module 200. In this case, the vibration transmission range becomes a peripheral region of the housing, and as the probe 1210 does not receive the external force when the vibration apparatus 10 is operated, durability of the vibration module 1000 during vibration may be improved and vibration of a target intensity may be continuously provided.

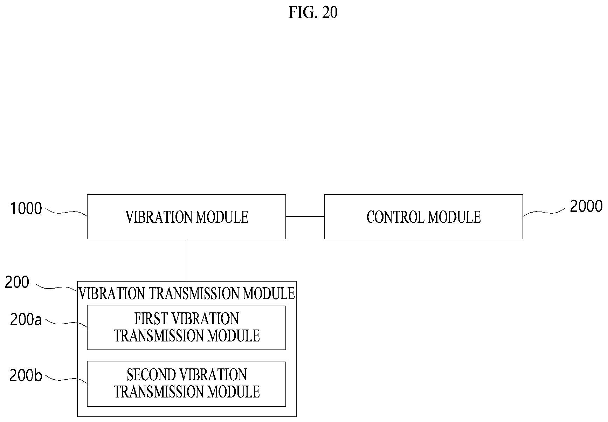

FIGS. 20 to 22 are diagrams for describing heights of the vibration transmission module 200 of the vibration apparatus 10. The heights of the vibration transmission module 200 of the vibration apparatus 10 will be described with reference to FIGS. 20 to 22.

The height of the vibration transmission module 200 may refer to a distance from a lower end of the vibration transmission module 200 to an upper end. For example, when a part protruding from each of the upper and lower parts of the vibration transmission module 200 is included, the height of the vibration transmission module 200 may refer to a distance from a part protruding furthest downward from the vibration transmission module 200 to a part protruding furthest upward.

Specifically, the height of the vibration transmission module 200 may be set in consideration of distances b1, b2, and b3 from a reference plane p to the upper end of the vibration transmission module 200 and a height a of the end of the probe 1210 according to various vibrations of the bobbin 1120. In order to induce dispersion of the external force applied to the probe 1210 to the vibration transmission module 200, the height of the vibration transmission module 200 may be set so that the distance b1 from the reference plane p to the end of the upper part of the vibration transmission module 200 is greater than the distance a from the virtual reference plan P perpendicular to the central axis of the bobbin 1120 to an end of the probe 1210 in a state in which the position of the bobbin 1120 is lowered to the maximum according to the vibration of the bobbin 1120.

In other words, the height of the vibration transmission module 200 may be provided so that the height a of the end of the probe 1210 during the maximum downward vibration of the bobbin 1120 does not exceed the distance b1 from the reference plane P to the upper end of the vibration transmission module 200 with respect to the virtual plane P having the vibration direction of the bobbin 1120 as a normal line and may induce dispersion of the external force applied to the probe 1210 to the vibration transmission module 200. That is, when the external force is simultaneously applied to the probe 1210 and the vibration transmission module 200 according to the operation of the vibration apparatus 10, a space R may be provided between the probe 1210 and the upper part of the vibration transmission module 200 so that the probe 1210 may vibrate downward, and thus, even when the external force acts on the probe 1210, the probe 1210 may continuously vibrate up and down.

Meanwhile, since the bobbin 1120 may be coupled to the elastic member 1140 and vibrate up and down together with the elastic member 1140, the position and state of the elastic member 1140 illustrated in FIGS. 20 to 22 may correspond to the position and state of the bobbin 1120.

Since there may be a difference in whether the probe 1210 is in contact with the human body or a degree of contact according to the height of the vibration transmission module 200, the height of the vibration transmission module 200 should be appropriately adjusted in relation to the probe 1210 according to the type and function of the vibration apparatus 10.

Hereinafter, the height of the vibration transmission module 200 in the case in which the shape of the vibration transmission module 200 is an open shape and the height of the vibration transmission module 200 in the case in which the shape of the vibration transmission module 200 is a closed shape will be described in more detail with reference to the drawings.

When the shape of the vibration transmission module 200 is an open shape, the vibration transmission module 200 may be provided to have a shape in which at least a part of the upper part is open.

Meanwhile, when the shape of the vibration transmission module 200 is an open shape, the vibration apparatus 10 may include a direct hitting type vibration apparatus 10 in which the probe 1210 directly hits the human body and an indirect hitting type vibration apparatus 10 in which the probe 1210 does not directly hit the human body.

Hereinafter, the height of the vibration transmission module 200 in the direct hitting type vibration apparatus 10 and the height of the vibration transmission module 200 in the indirect hitting type vibration apparatus 10 will be described in more detail with reference to the drawings.

The vibration apparatus 10 may be an indirect hitting type vibration apparatus 10 in which the probe 1210 does not directly hit the human body when the shape of the vibration transmission module 200 is an open shape.

FIGS. 20A to 20C are diagrams illustrating changes in position of the probe 1210 according to the vibration of the vibration apparatus 10. In this case, FIG. 17A illustrates a state in which the elastic member 1140 is contracted to the maximum, FIG. 17C illustrates a state in which the elastic member 1140 is relaxed to the maximum, and FIG. 17B illustrates an intermediate state of FIG. 17A and FIG. 17C.

In FIGS. 20A to 20C, the distance between the virtual plan P and the vibration transmission module 200 may be changed according to the changes in position of the probe 1210. Accordingly, the distance between the vibration transmission module 200 and the end of the probe 1210 may also be changed. That is, the distance between the vibration transmission module 200 and the end of the probe 1210 may be the longest in FIG. 17 A and may be shortest in FIG. 17C.

In the case of the indirect hitting type vibration apparatus 10, the height of the vibration transmission module 200 may be set so that the distance b3 from the virtual plan P to an end of the upper part of the vibration transmission module 200 is greater than the distance a from the virtual plan P perpendicular to the central axis of the bobbin 1120 to an end of the probe 1210 in the state in which the position of the bobbin 1120 is increased to the maximum due to the vibration of the bobbin 1120, as illustrated in FIG. 17C, in order to achieve indirect vibration transmission by the probe 1210.

That is, even in the state in which the position of the bobbin 1120 is increased to the maximum due to the vibration of the vibration apparatus 10, the probe 1210 may not protrude to the outside of the vibration apparatus 10. For example, the probe 1210 may be provided so as not to protrude to the outside of the upper part of the vibration transmission module 200 in a section in which the vertical reciprocating movement of the bobbin 1120 is repeated at least once or more according to the operation of the vibration apparatus 10. Accordingly, when the vibration apparatus 10 vibrates, the external force may not act on the probe 1210, and accordingly, the vibration generated by the vibration apparatus 10 may be transmitted only through the vibration transmission module 200.

Meanwhile, when the shape of the vibration transmission module 200 is an open shape, the vibration apparatus 10 may be a direct hitting type vibration apparatus 10 in which the probe 1210 directly hits the human body.

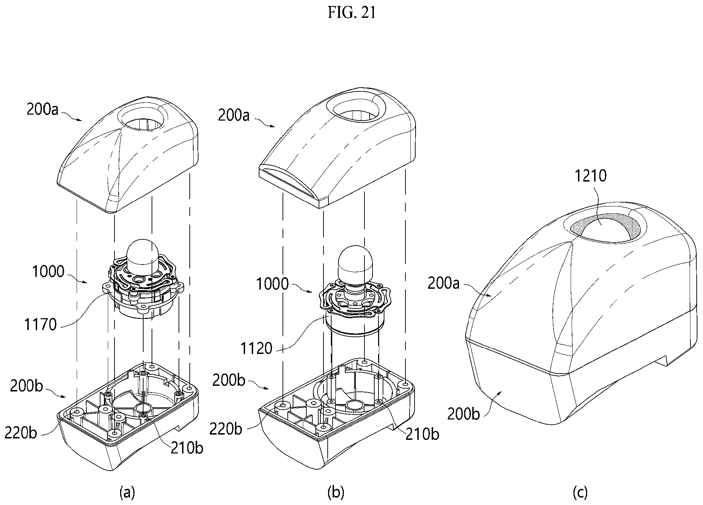

FIGS. 21A to 21C are diagrams illustrating changes in position of the probe 1210 according to the vibration of the vibration apparatus 10. In this case, FIG. 18A illustrates a state in which the elastic member 1140 is contracted to the maximum, FIG. 18C illustrates a state in which the elastic member 1140 is relaxed to the maximum, and FIG. 18B illustrates an intermediate state of FIG. 18A and FIG. 18C.

Referring to FIG. 18, in the case of the direct hitting type vibration apparatus 10, the height of the vibration transmission module 200 may be set so that the distance b3 from the virtual plan to an end of the upper part of the vibration transmission module 200 is smaller than the distance a from the virtual plan P perpendicular to the central axis of the bobbin 1120 to an end of the probe 1210 in the state in which the position of the bobbin 1120 is increased to the maximum due to the vibration of the bobbin 1120, as illustrated in FIG. 18C, in order to achieve direct vibration transmission by the probe 1210.

In this case, the probe 1210 may be provided so that at least a part of the probe 1210 protrudes to the outside of the upper part of the vibration transmission module 200 in a section in which the vertical reciprocating movement of the bobbin 1120 is repeated at least once or more according to the operation of the vibration apparatus 10. Accordingly, when the vibration apparatus 10 vibrates, the vibration may be simultaneously transmitted by the probe 1210 and the vibration transmission module 200, and thus a wider vibration transmission range may be secured. Further, even when the external force is applied to the probe 1210 when the vibration apparatus 10 vibrates, the probe 1210 may vibrate in a space R between the probe 1210 and the vibration transmission module 200, and thus continuous vibration transmission may be achieved.

When the shape of the vibration transmission module 200 is a closed shape, the vibration transmission module 200 may be provided in a shape in which the upper part is closed.

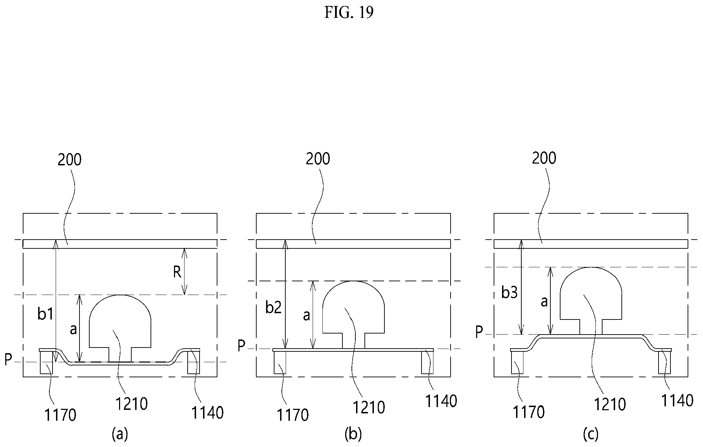

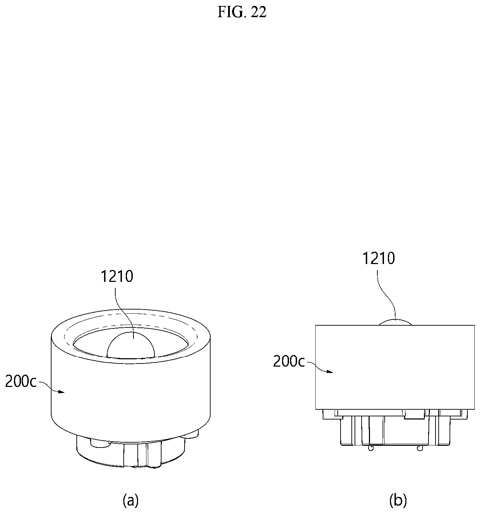

FIGS. 22A to 22C are diagrams illustrating changes in position of the probe 1210 according to the vibration of the vibration apparatus 10. In this case, FIG. 19A illustrates a state in which the elastic member 1140 is contracted to the maximum, FIG. 19C illustrates a state in which the elastic member 1140 is relaxed to the maximum, and FIG. 19B illustrates an intermediate state of FIG. 19A and FIG. 19C.

When the shape of the vibration transmission module 200 is a closed shape, the height of the vibration transmission module 200 may be set so that the distance b3 from the virtual plan to an end of the upper part of the vibration transmission module 200 is greater than the distance a from the virtual plan P perpendicular to the central axis of the bobbin 1120 to an end of the probe 1210 in the state in which the position of the bobbin 1120 is increased to the maximum due to the vibration of the bobbin 1120, as illustrated in FIG. 19C, in order to achieve indirect vibration transmission by the probe 1210.

For example, the probe 1210 may be provided so as not to protrude to the outside of the upper part of the vibration transmission module 200 in a section in which the vertical reciprocating movement of the bobbin 1120 is repeated at least once or more according to the operation of the vibration apparatus 10. Accordingly, when the vibration apparatus 10 vibrates, the external force does not act on the probe 1210, and thus the vibration generated by the vibration apparatus 10 may be transmitted only through the vibration transmission module 200 so that indirect vibration may be provided. Further, when the vibration apparatus 10 vibrates, the external force does not act on the probe 1210, and thus the durability of the probe 1210 may be enhanced and vibration of a target intensity may be continuously provided regardless of external pressure applied to the vibration apparatus 10.

FIG. 20 is a block diagram for describing a type of a vibration transmission module.

Referring to FIG. 20, a vibration transmission module 200 of a vibration apparatus 10 according to the embodiment of the present invention may include a first vibration transmission module 200a formed to surround at least a part of the probe 1210, and a second vibration transmission module 200b formed to surround at least a part of the housing 1170.

FIG. 21 illustrates diagrams for describing a type of a vibration transmission module and a relationship between the vibration transmission module and a vibration module. Hereinafter, a type of the vibration transmission module 200 and a relationship between the vibration transmission module 200 and the vibration module 1000 will be described in more detail with reference to FIG. 21.

Referring to FIG. 21A, a first vibration transmission module 200a may serve to protect a probe 1210 while surrounding at least a part of the probe 1210 and not being in contact with the probe 1210. That is, the first vibration transmission module 200a may have an inner space, at least a part of the probe 1210 may be located in the inner space, and the first vibration transmission module 200a may be provided to surround the at least a part of the probe 1210 and not to be in contact with the probe 1210.

A second vibration transmission module 200b may surround at least a part of the vibration module 1000 and be in contact with the at least a part of the vibration module 1000. Specifically, the second vibration transmission module 200b may surround at least a part of the housing 1170 and be in contact with the at least a part of the housing 1170. That is, the second vibration transmission module 200b may have an inner space and at least a part of the housing 1170 may be located in the inner space.

The first vibration transmission module 200a may be coupled to the second vibration transmission module 200b. That is, the first vibration transmission module 200a and the second vibration transmission module 200b may include the vibration module 1000 in at least a part of the inner space thereof and be coupled to each other.

A process in which vibration generated by the vibration module 1000 is transmitted to the vibration transmission module 200 will be described in detail.

Referring to a method of transmitting vibration generated by the vibration module 1000, when vibration is generated by the vibration module 1000, the vibration may be transmitted from the vibration module 1000 to the second vibration transmission module 200b and then transmitted to the first vibration transmission module 200a coupled to the second vibration transmission module 200b. As a result, the vibration generated by the vibration module 1000 may not be directly transmitted to the first vibration transmission module 200a. Further, the vibration generated by the probe 1210 of the vibration module 1000 may not be directly transmitted to the first vibration transmission module 200a. This is because the second vibration transmission module 200b is brought into contact with at least a part of the vibration module 1000, whereas the first vibration transmission module 200a is coupled to the second vibration transmission module 200b while surrounding at least a part of the probe 1210 and not being in contact with the probe 1210. The vibration generated by the vibration module 1000 may be transmitted to the first vibration transmission module 200a through the second vibration transmission module 200b, and thus the vibration apparatus 10 may provide the vibration in a wider range.

Meanwhile, the first vibration transmission module 200a may be provided to have an open shape in which at least a part of an upper part is open. Further, the first vibration transmission module 200a may be provided to have a closed shape in which the upper part is closed. Since the case in which the shape of the first vibration transmission module 200a is an open shape and the case in which the shape of the first vibration transmission module 200a is a closed shape correspond to the case in which the shape of the vibration transmission module 200 is an open shape and the case in which the shape of the vibration transmission module 200 is a closed shape, respectively, as described above, the descriptions thereof will not be repeated.

The second vibration transmission module 200b may include a plurality of coupling members 210b that can be coupled to at least a part of the housing 1170. That is, the second vibration transmission module 200b may be formed to surround the at least a part of the housing 1170 and may be coupled to the at least a part of the housing 1170. For example, the plurality of coupling members 210b may have a cylindrical shape, and an inside of the cylindrical shape may include a screw groove so as to be screwed with the connecting part 1173 of the housing 1170 described above.

More specifically, as described above, the housing 1170 may include the body part 1172 and the connecting part 1173, and in order to transmit the vibration generated by the vibration module 1000 to the second vibration transmission module 200b, the plurality of coupling members 210b of the second vibration transmission module 200b may be coupled to the connecting part 1173 of the housing 1170.

In this case, when the plurality of coupling members 210b of the second vibration transmission module 200b are coupled to the connecting part of the housing 1170, the second vibration transmission module 200b may be disposed to be spaced apart from the body part 1172 of the housing 1170. In other words, the second vibration transmission module 200b may be provided so as to be coupled to the connecting part 1173 of the housing 1170 but not to be in contact with the body part 1172 of the housing 1170. That is, the second vibration transmission module 200b may be coupled to the connecting part 1173 of the housing 1170 but may be present to be spaced apart from the body part 1172 of the housing 1170. Further, the second vibration transmission module 200b may be formed so as not to be in contact with the vibration generating unit 1100 of the vibration module 1000 described above.

For example, the second vibration transmission module 200b may be formed to be spaced apart from the housing 1170 so as not to be in contact with a lower surface of the housing 1170 when the housing 1170 is moved up and down according to the operation of the vibration module 1000.

Meanwhile, the first vibration transmission module 200a may have a structure that can be coupled to the second vibration transmission module 200b. For example, the first vibration transmission module 200a and the second vibration transmission module 200b may be detachably coupled.

Meanwhile, referring to FIG. 21B, the second vibration transmission module 200b may be integrally formed with the housing 1170 of the vibration generating unit 1100. The second vibration transmission module 200b may be integrally formed with the housing 1170 and may perform a function of the housing 1170. For example, the second vibration transmission module 200b may provide a space for accommodating the magnet 1110, the bobbin 1120, the coil 1130, and the like. In other words, when the second vibration transmission module 200b is integrally formed with the housing 1170 of the vibration generating unit 1100, at least one of the plurality of coupling members 210b of the second vibration transmission module 200b may be coupled to at least one of the damper coupling holes formed in the damper d. In this case, the second vibration transmission module 200b may be formed to be spaced apart from the bobbin 1120 so as not to be in contact with the lower surface 1123 of the bobbin 1120 when the bobbin 1120 is moved up and down according to the operation of the vibration module 1000. In this specification, as in the above embodiment, a component of the second vibration transmission module 200b, which is integrally formed with the housing 1170 of the vibration generating unit 1100, may be expressed as the second vibration transmission module 200b or as the housing 1170.

Since the second vibration transmission module 200b is integrally formed with the housing 1170, a manufacturing process of the vibration apparatus 10 may be simplified and thus a manufacturing cost may be reduced. Further, since the second vibration transmission module 200b is integrally formed with the housing 1170, a size of the vibration apparatus 10 may be further reduced.

When the second vibration transmission module 200b is integrally formed with the housing 1170, the vibration apparatus 10 may transmit the vibration generated by the vibration generating unit 1100 to the vibration receiving unit S through the vibration transmission module 200 or the housing 1170.

FIG. 22 illustrates diagrams for describing a vibration transmission module according to another embodiment.

Referring to FIG. 22, the first vibration transmission module 200a and the second vibration transmission module 200b may include a third vibration transmission module 200c that is integrally formed. Since the first vibration transmission module 200a and the second vibration transmission module 200b are integrally formed, durability of the vibration transmission module 200 may be maintained even when the vibration of the vibration module 1000 lasts for a long time.

According to an embodiment, an application may include a vibration module 1000, a control module 2000, a vibration transmission module 200, and a vibration receiving unit S. In this case, as described above, the application may include a massage device or an exercise apparatus.

Hereinafter, the application including the massage device and the exercise apparatus will be described with reference to the drawings. However, as described above, since the vibration module 1000 and the control module 2000 included in the application correspond to the vibration module 1000 and the control module 2000 described above with reference to FIG. 1, the same reference numerals may be assigned to common parts and detailed descriptions thereof will be omitted.

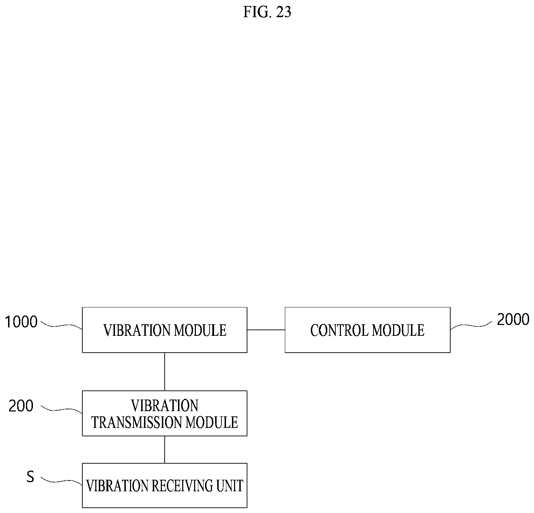

FIG. 23 is a block diagram for describing an application including a vibration apparatus.

Referring to FIG. 23, the application according to the embodiment may include a vibration receiving unit S. The vibration receiving unit S may enable the vibration generated by the vibration module 1000 of the application to be spread uniformly over main parts of the application. That is, the vibration receiving unit S may perform a function of uniformly transmitting the vibration to an entirety of the human body that is brought into contact with the application.

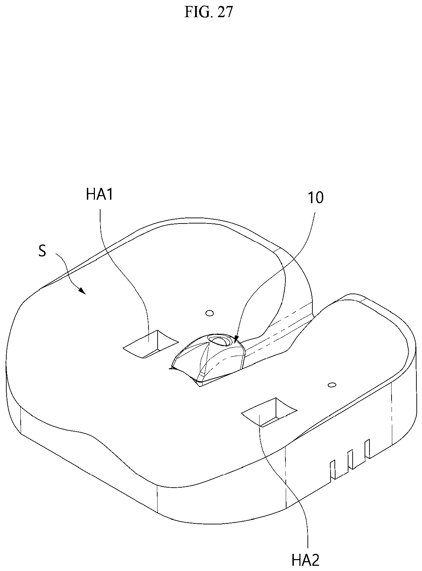

FIGS. 27 to 29 are diagrams for describing the vibration receiving unit S of the application.

The vibration receiving unit S of the application may be provided to have a shape suitable for the purpose and function of the application.

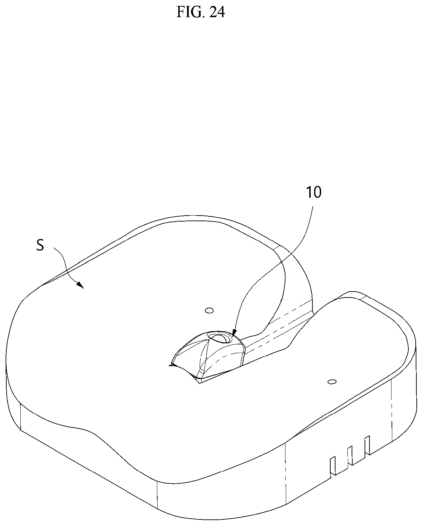

Referring to FIG. 24, when the application is a massage device, the vibration receiving unit S may be provided to have a shape corresponding to a target body part to be massaged. For example, the vibration receiving unit S of the massage device may have a cushion shape. More specifically, when the massage device performs a function of massaging a perineal region, the vibration receiving unit S may have a cushion shape. In this case, when the vibration receiving unit S is provided to have a cushion shape, the cushion shape may include various known cushion shapes.

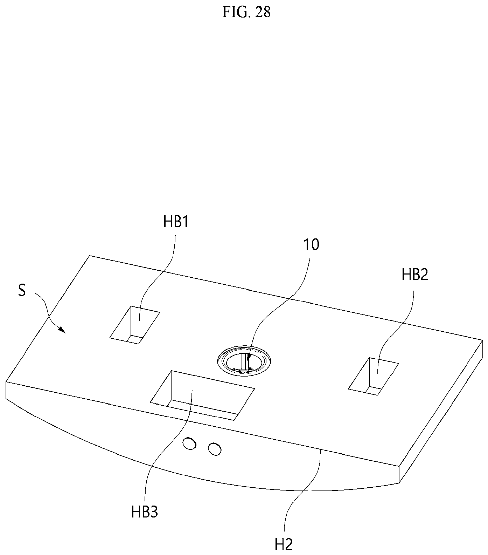

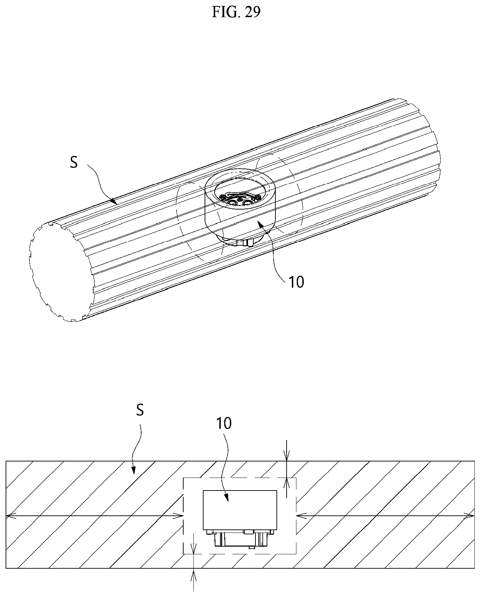

Referring to FIGS. 28 and 29, when the application is an exercise apparatus, the vibration receiving unit S may be provided to have a shape corresponding to a target human body part on which the exercise apparatus is intended to perform its function.

For example, the vibration receiving unit S of the exercise apparatus may have a cylindrical shape. As another example, the vibration receiving unit S of the exercise apparatus may have a spherical shape.

More specifically, when the exercise apparatus is used as a foam roller, the vibration receiving unit S may have a cylindrical shape or a spherical shape. In this case, the cylindrical shape or the spherical shape may include various known foam roller shapes.

As another example, the vibration receiving unit S of the exercise apparatus may be formed in the form of a balance cushion. That is, the vibration receiving unit S of the exercise apparatus may have an upper part having a flat plate shape and a lower part having a shape of which a center is convex so that the user may climb on the upper part of the vibration receiving unit S and perform an exercise to maintain a balance in left and right directions.

Further, the vibration receiving unit S may be provided to have a shape surrounding at least a part of the vibration module 1000 and the vibration transmission module 200. For example, the vibration receiving unit S may be provided to have a shape surrounding side surfaces of the vibration module 1000 and the vibration transmission module 200.

Further, the vibration receiving unit S may be formed to be in direct or indirect contact with at least one of the vibration module 1000 or the vibration transmission module 200. For example, the vibration receiving unit S may be formed to be in direct contact with the at least one of the vibration module 1000 or the vibration transmission module 200 and may more directly receive the vibration transmitted from the vibration module 1000 and the vibration transmission module 200. As another example, the vibration receiving unit S may be formed to be in indirect contact with the at least one of the vibration module 1000 or the vibration transmission module 200 and may receive the vibration in a state in which the vibration transmitted from the vibration module 1000 and the vibration transmission module 200 is further reduced.

Since various materials including a material used for a known cushion may be used as a material of the vibration receiving unit S provided in the massage device, specific descriptions thereof will be omitted. For example, the material of the vibration receiving unit S provided in the massage device may be a sponge. As another example, the vibration receiving unit S provided in the massage device may be made of a material having elasticity.

Further, since various materials including a material used for a known foam roller may be used as the material of the vibration receiving unit S provided in the exercise apparatus, specific descriptions thereof will be omitted. For example, the material of the vibration receiving unit S provided in the exercise apparatus may be ethylene vinyl acetate copolymer (EVA) or expanded polypropylene (EPP). As another example, the vibration receiving unit S provided in the exercise apparatus may be made of a material having elasticity.

FIG. 27 is a diagram for describing a vibration receiving unit of a massage device in which a vibration module is disposed, and FIG. 28 is a diagram for describing an exercise apparatus in which a vibration module is disposed.

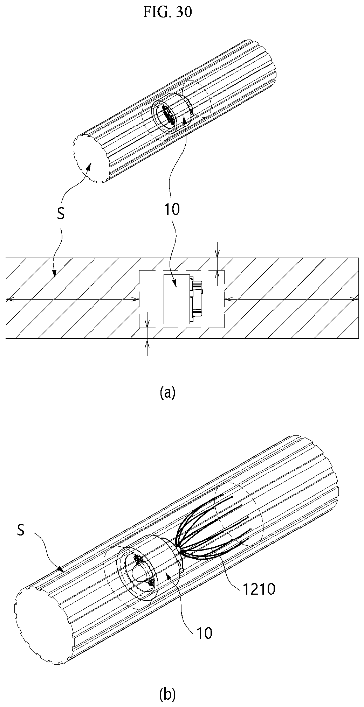

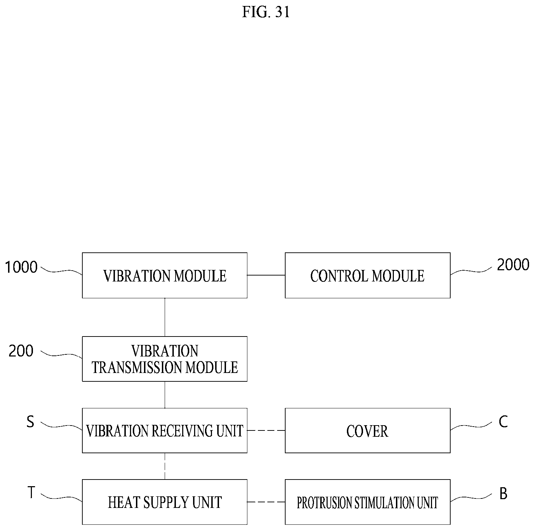

An arrangement position, number, and direction of the vibration module 1000 disposed in the vibration receiving unit S will be described with reference to FIGS. 30 and 31.

The vibration module 1000 of the application according to an embodiment may be disposed in a central region of the vibration receiving unit S. As another example, the vibration module 1000 may be disposed to be laterally symmetrical with respect to the central region of the vibration receiving unit S. More specifically, the vibration module 1000 may be disposed in the vibration receiving unit S and may be disposed in a part of the vibration receiving unit S corresponding to the human body part that requires more intensive vibration stimulation when the application is operated.

Further, the vibration module 1000 may be disposed in the vibration receiving unit S and may be disposed in an unfixed state so that an arrangement position, direction, and number may be freely changed.

Referring to FIG. 27, at least one vibration module 1000 may be disposed in the vibration receiving unit S of the massage device according to the embodiment. For example, only one vibration module 1000 may be disposed in the central region of the vibration receiving unit S or a plurality of vibration modules 1000 may be disposed.

In the massage device, the vibration module 1000 may be disposed in the central region of the vibration receiving unit S so that the strongest vibration may be applied to a perineal region, and, at the same time, the vibration may be transmitted to the vibration receiving unit S so that the vibration may be transmitted to the body in contact with the vibration receiving unit S, for example, a leg, a thigh, etc. Accordingly, by stimulating the perineal region, the strong vibration may be transmitted to the intestine to provide excellent effects even for constipation.

As another example, the vibration receiving unit S of the massage device may be additionally disposed on any one of parts HA1 and HA2 of the human body that the thigh touches. As still another example, by arranging a speaker in any one of openings HA1 and HA2 formed in the vibration receiving unit S of the massage device 210, the same sound source as the vibration generated by the vibration apparatus 10 may be output through the speaker and the beneficial effect of the vibration may be further improved. In this case, the sound source used in the vibration apparatus 10 and the sound source output through the speaker may be different.

Referring to FIG. 28, in the vibration receiving unit S of the exercise apparatus according to an embodiment, the vibration module 1000 may be disposed in the central region of the vibration receiving unit S. As another example, the vibration module 1000 may be disposed to be laterally symmetrical with respect to the central region of the vibration receiving unit S. More specifically, the vibration module 1000 may be disposed in the vibration receiving unit S and may be disposed in a part of the vibration receiving unit S corresponding to the human body part that requires more intensive vibration stimulation when the exercise apparatus is operated.

Further, at least one vibration module 1000 may be disposed in the vibration receiving unit S of the exercise apparatus. That is, only one vibration module 1000 may be disposed in the central region of the vibration receiving unit S or a plurality of vibration modules 1000 may be disposed.

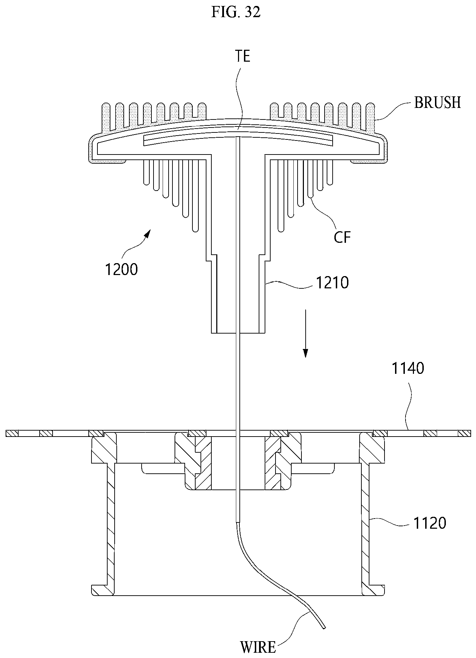

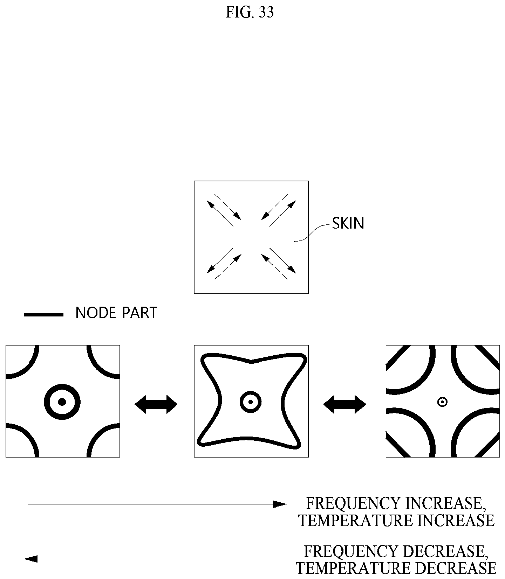

FIGS. 32 and 33 are diagrams for describing arrangement directions of the vibration module disposed in the vibration receiving unit.

Referring to FIG. 29, at least one vibration module 1000 may be disposed in the vibration receiving unit S and may be disposed so that the vibration direction of the probe 1210 corresponds to a height direction of the vibration receiving unit S. In this case, a height direction of the vibration receiving unit S refers to a direction perpendicular to a surface with respect to an upper or lower surface of the vibration receiving unit S. More specifically, the height direction of the vibration receiving unit S may refer to a vertical direction of the vibration receiving unit S. For example, the vibration module 1000 may be disposed so that a direction in which a distance between the outer surface of the vibration receiving unit S and one surface of the vibration transmission module 200 is minimized and a vibration direction of the vibration module 1000 match.

As a result, the vibration module 1000 may be disposed in the vibration receiving unit S so that the probe 1210 faces the height direction of the vibration receiving unit S. Accordingly, the vibration generated by the vibration module 1000 may be transmitted to the user at the shortest distance, and thus the vibration generated by the vibration module 1000 may be effectively transmitted to the body of the user of the massage device.

Referring to FIG. 30, at least one vibration module 1000 may be disposed in the vibration receiving unit S and may be disposed so that the vibration direction of the probe 1210 corresponds to a longitudinal direction of the vibration receiving unit S. More specifically, the vibration module 1000 may be disposed so that a direction in which a distance between the outer surface of the vibration receiving unit S and one surface of the vibration transmission module 200 is maximized and a vibration direction of the vibration module 1000 match.

FIG. 31 is a block diagram for describing additional functions provided by the application. Referring to FIG. 31, the application may include a cover C, a heat supply unit T, a protrusion stimulation unit B, and the like in addition to the vibration module 1000, the control module 2000, the vibration transmission module 200, and the vibration receiving unit S.

The cover C may be formed to have a shape corresponding to the shape of the vibration receiving unit S and may cover an outer surface of the vibration receiving unit S. Further, the heat supply unit T may perform a function of further maximizing a massage and/or exercise effect by transmitting heat to the human body in contact with the application. Further, the protrusion stimulation unit B may be formed on the outer surface of the vibration receiving unit S or the cover C and may additionally transmit stimulation by the protrusion to the user who uses the application, and thus the stimulation effect by vibration may be maximized.

Hereinafter, the acoustic wave vibration apparatus 100 performing a specific function will be described with reference to FIGS. 36 and 37.

FIG. 32 is a diagram illustrating a vibration amplification unit 1200 for performing a cooling and heating function and a vibration function according to an embodiment of this specification. Hereinafter, the acoustic wave vibration apparatus 100 is mainly described as being used for skin care such as cleansing, washing, and applying lotion, but the scope of the present invention is not limited thereto.

Referring to FIG. 32, the vibration amplification unit 1200 may include a probe 1210, a brush, a temperature control member TE, and a heat dissipation member CF. A temperature of the vibration amplification unit 1200 may be adjusted by the temperature control member TE, heat may be emitted through the heat dissipation member CF, and vibration may be provided to the skin through the brush.

The brush may be brought into contact with the skin to provide the vibration or apply a skin care product. Here, the brush may include a plurality of protrusions in order to increase a contact surface with the skin. In this case, the shape of the protrusion may include a conical shape, a truncated conical shape, a pyramid shape, a truncated pyramid shape, a cylindrical shape, a hemispherical shape, a prismatic shape (a triangular prism with a rectangular or square base), a truncated prismatic shape, a cubic shape, a pentahedral shape (a base of which has a rectangular shape), a truncated pentahedral shape, and deformations or modifications thereof.

The brush may be made of a thermoplastic or thermosetting polymer composition. For example, the brush may be made of at least one of silicone rubber, rubber polyurethane, styrene-butadiene rubber (SBR), butyl rubber (isobutyleneisoprene copolymer), natural or synthetic polyisoprene, nitrile rubber (butadieneacrylonitrile rubber), rubber polypropylene, ethylene propylene diene copolymer (EPDM), ethylene propylene copolymer (EPM), and mixtures thereof. The brush may be made of a thermoplastic or thermosetting polymer composition, and thus it is possible to alleviate skin irritation caused by vibration.

The brush may be attached to or detached from the vibration amplification unit 1200. For example, the brush may be elastically deformed and covered on the upper end of the probe 1210 and may be removed from the probe 1210 for cleaning or replacement after skin care.

As described above, when the acoustic wave vibration apparatus 100 provides the vibration to the skin, the brush may alleviate irritation to the skin while increasing the contact surface with the skin and thus the skin care effect may be improved.

The temperature control member TE may perform a cooling and heating function on the skin through the probe 1210. The temperature control member TE may provide cooling energy or heat energy. For example, the temperature control member TE may receive a control signal of the control module 2000 and provide cooling energy or heat energy to the probe 1210, and thus a temperature of a part of the probe 1210 in contact with the skin may be increased or decreased.

The temperature control member TE may utilize a thermoelectric effect. For example, the temperature control member TE may include a thermoelectric element that uses heat absorption or heat generation by the Peltier effect. Specifically, in the temperature control member TE, one terminal may absorb heat and the other terminal may generate heat, a terminal that absorbs heat and a terminal that generates heat in a direction of a current applied may be switched, and an amount of heat absorption and heat generation may be adjusted according to an amount of applied current. Here, the temperature control member TE may include a flexible thermoelectric element having elasticity. In addition, here, the temperature control member TE may include a flat thermoelectric element. Hereinafter, for convenience of description, the case is described in which the temperature control member TE is a thermoelectric element, but the scope of the present invention is not limited thereto.

The temperature control member TE may be disposed inside the vibration amplification unit 1200. For example, as illustrated in FIG. 32, the temperature control member TE may be disposed inside the probe 1210, and at least one surface of the temperature control member TE may be in contact with at least a part of an inner surface of the probe 1210. Specifically, an upper surface of the temperature control member TE may be in surface contact with a part of the inner surface of the probe 1210 that is in contact with the skin. Alternatively, the lower surface of the temperature control member TE may be in surface contact with a part of the inner surface of the probe 1210 that corresponds to a part to which a heat dissipation fin CF to be described below is attached. At least a part of the temperature control member TE may be in surface contact with at least a part of the inner surface of the probe 1210, and thus heat transfer efficiency may be improved.

The vibration amplification unit 1200 may include a hollow therein for electrical connection of the temperature control member TE. For example, as illustrated in FIG. 32, the probe 1210 may include a hollow therein, and a cord for electrically connecting the temperature control member TE to the control module 2000 may be inserted into the probe 1210. Further, the cord may pass through a central hole of the elastic member 1140 and the bobbin 1120 into which the vibration amplification unit 1200 is inserted and may be connected to the control module 2000 and thus may electrically connect the temperature control member TE to the control module 2000.

Meanwhile, the vibration amplification unit 1200 may be implemented as an assembly type to include the temperature control member TE therein. For example, the probe 1210 may be divided into a first probe member in contact with the skin and a second probe member connected to the bobbin 1120 for a manufacturing process. In this case, the first probe member and the second probe member may include different configurations. Alternatively, the first probe member and the second probe member may be connected through an insulating member to be thermally separated.

The heat dissipation fin CF may discharge the heat in the vibration amplification unit 1200 to the outside. For example, the heat dissipation fin CF may receive the heat generated by the temperature control member TE inside the probe 1210 and discharge the heat to the outside. Specifically, when the temperature control member TE performs a cooling function on the skin, heat energy may be generated on the heating surface of the temperature control member TE, and the heat dissipation fin CF may receive the heat energy and discharge the heat energy to the outside by being in contact with the outside air in order to prevent a decrease in cooling efficiency due to the heat energy.

The heat dissipation fin CF may be implemented in various shapes. For example, as illustrated in FIG. 32, the heat dissipation fin may be implemented to have a rectangular parallelepiped shape or a cylindrical shape in order to increase the contact surface with the outside air. Meanwhile, an end of the heat dissipation fin may be implemented to have a flat or convex shape for user safety.

The vibration amplification unit 1200 may include a plurality of heat dissipation fins CF. For example, as illustrated in FIG. 32, the plurality of heat dissipation fins CF may be provided on a part of the outer surface of the probe 1210 corresponding to the heating surface of the temperature control member TE. In this case, the plurality of heat dissipation fins CF may be provided in various directions such as a direction perpendicular to the surface of the probe 1210, an inclined direction, and the like. As another example, the heat dissipation fin CF may be directly connected to the temperature control member TE. As described above, the heat dissipation fin CF may receive the heat directly from the temperature control member TE or may receive the heat indirectly through the probe 1210 and discharge the heat to the outside of the vibration amplification unit 1200.

Hereinafter, a method in which the acoustic wave vibration apparatus 100 simultaneously performs a cooling and/or heating function and a vibration function will be described with reference to FIG. 33.

FIG. 33 is a diagram illustrating a change in skin condition according to a vibration frequency according to an embodiment of this specification.

Referring to FIG. 33, when a certain target receives acoustic wave vibration, a part vibrating in a specific region of the target may be specified according to a vibration frequency. Specifically, the acoustic wave vibration for the target may be provided as a stationary wave and accordingly, a non-vibrating node part may be present in the specific region of the target. For example, as illustrated in FIG. 33, when the skin receives acoustic wave vibration, the skin may include a non-vibrating node part of a specific pattern according to the vibration frequency.

The pattern of the node part described above may be modified according to the frequency. For example, as illustrated in FIG. 33, as the frequency increases, the pattern of the node part may be changed to a different pattern. Specifically, as the vibration frequency increases, the node part may be moved from the center to the peripheral part. Alternatively, as the vibration frequency decreases, the node part may be moved from the peripheral part to the center. Hereinafter, the acoustic wave vibration apparatus 100 for effectively providing a skin care method using the pattern change of the node part and the cooling and heating function will be described.

The control module 2000 may change the vibration frequency while the vibration module 1000 is operated. For example, the control module 2000 may change the vibration frequency by controlling the vibration generating unit 1100 while the vibration module 1000 provides vibration energy to the skin. Specifically, the control module 2000 may provide a control signal to the vibration generating unit 1100 to have a frequency that is changed at a preset speed within a preset frequency range for a preset time.

Here, the preset frequency range in which the vibration frequency is changed may be variously set. For example, the preset frequency range may include a range in which the pattern of the node part is changed according to the above-described acoustic wave vibration. Specifically, the preset frequency range may include a frequency range in which the pattern is changed based on a Chladni pattern. More specifically, the preset frequency range may be set in at least some of a range of 150 Hz to 350 Hz, a range of 250 Hz to 500 Hz, a range of 400 Hz to 750 Hz, and a range of 650 Hz to 950 Hz. In this case, the size of the preset frequency range may be set at 200 Hz or lower.

In addition, here, the preset time at which the vibration frequency is changed may be variously set. For example, the preset time may be set as a time corresponding to skin care such as washing, cleansing, and the like. Specifically, the preset time may be set to a time between five seconds to 30 seconds.

In addition, here, the preset speed may be variously set according to the skin care method provided by the acoustic wave vibration apparatus 100. For example, the preset speed may be set based on the preset time and the preset frequency range described above. In this case, the preset speed may be maintained at the same speed for the preset time or may be changed.

As described above, the control module 2000 may change the vibration pattern of the skin surface by changing the frequency of the vibration module 1000 and thus may provide stimulation in a direction parallel to the skin surface. For example, the control module 2000 may perform a function of enlarging the pores located in the center of the skin surface by gradually increasing the frequency of the vibration module 1000 to move a non-vibrating part of the skin on the skin surface in contact with the vibration module 1000 in a direction away from the center of the skin surface. As another example, the control module 2000 may perform a function of reducing the pores located in the center of the skin surface by gradually reducing the frequency of the vibration module 1000 to move a non-vibrating node part on the skin surface in contact with the vibration module 1000 in a direction closer to the center of the skin surface.

Here, the control module 2000 may provide cooling energy or heat energy to the skin while the pores of the skin are reduced or enlarged by the vibration module 1000. In other words, the preset frequency range, the preset time, and the preset speed described above may be set based on the control of the temperature control member TE to be described below.

The control module 2000 may change the temperature of the vibration amplification unit 1200. For example, the control module 2000 may induce an endothermic or exothermic reaction by applying an electric signal to the temperature control member TE, thereby increasing or decreasing the temperature of the probe 1210 and thus may maintain or change the temperature of the probe 1210 within the preset temperature range.

The control module 2000 may control the vibration frequency of the vibration module 1000 and, at the same time, control the temperature of the vibration amplification unit 1200. Hereinafter, the above description may be applied without change to a method in which the control module 2000 controls the frequency of the vibration module 1000.

For example, the control module 2000 may increase the temperature of the vibration amplification unit 1200 while increasing the vibration frequency of the vibration module 1000. Specifically, the control module 2000 may increase the temperature of the probe 1210 to a preset temperature higher than room temperature using the temperature control member TE while increasing the vibration frequency of the vibration module 1000 to a preset speed for a preset time within a preset range. Here, the control module 2000 may increase the temperature of the probe 1210 before, after, or during the change of the vibration frequency of the vibration module 1000. In this case, the preset temperature may be set within a range of 30 °C to 60 °C.

As illustrated in FIG. 33, the control module 2000 may increase the temperature of the vibration amplification unit 1200 while increasing the vibration frequency of the vibration module 1000, and thus skin pores may be effectively enlarged and the skin may be relaxed, and skin flow is activated so that skin beauty products may be absorbed more actively.

As another example, the control module 2000 may reduce the temperature of the vibration amplification unit 1200 while reducing the vibration frequency of the vibration module 1000. Specifically, the control module 2000 may reduce the temperature of the probe 1210 to a preset temperature lower than room temperature using the temperature control member TE while reducing the vibration frequency of the vibration module 1000 to a preset speed for a preset time within a preset range. Here, the control module 2000 may reduce the temperature of the probe 1210 before, after, or during the change of the vibration frequency of the vibration module 1000. In this case, the preset temperature may be set within a range of -15 °C to 25 °C.

As illustrated in FIG. 33, the control module 2000 may reduce the temperature of the vibration amplification unit 1200 while reducing the vibration frequency of the vibration module 1000, and thus skin pores may be effectively contracted to soothe the skin after cleansing and prevent the penetration of waste.

As still another example, the control module 2000 may reduce the temperature of the vibration amplification unit 1200 while increasing the vibration frequency of the vibration module 1000 or may increase the temperature of the vibration amplification unit 1200 while reducing the vibration frequency of the vibration module 1000.

The control module 2000 may control the vibration frequency of the vibration module 1000 and the temperature of the vibration amplification unit 1200 step by step. For example, the control module 2000 may increase or reduce the vibration frequency and the temperature of the vibration amplification unit 1200, and then reduce or increase the vibration frequency and the temperature of the vibration amplification unit 1200 again.

Meanwhile, the acoustic wave vibration apparatus 100 may perform an additional function in addition to the above-described cooling and heating function or vibration function. For example, the acoustic wave vibration apparatus 100 may perform a galvanic skin care function. Here, the galvanic skin care may refer to a skin care method in which skin beauty products such as cosmetics are efficiently absorbed into the skin using a minute electric current. Specifically, skin beauty products such as cosmetics having a specific polarity may be applied to the skin and a galvanic current having the same polarity may be applied to the skin to penetrate the skin beauty products deep into the skin.

For the above-described functions, the acoustic wave vibration apparatus 100 may include at least one electrode having a specific polarity according to the electric signal provided. For example, the vibration amplification unit 1200 may include an electrode that receives an electric signal and generates a galvanic current having a specific polarity. Specifically, at least a part of the probe 1210 may include an electrode, and the control module 2000 may generate a galvanic current by providing an electric signal to the electrode.

Further, the above-described functions may be performed simultaneously with or before or after performing the cooling and heating function or the vibration function of the acoustic wave vibration apparatus 100. For example, the user may perform a galvanic skin care function using the acoustic wave vibration apparatus 100 before cleansing, wash his or her face using the cooling and heating function and the vibration function, and then perform the galvanic skin care function again to supply nutrients to the skin.

According to the embodiments of the present invention, by providing vibration using acoustic pressure to the user, the user can receive a soft and cool massage feeling.

According to the embodiments of the present invention, a plurality of points at which a damper of an elastic member that performs a vibration function in a vibration module is bent can be provided, and thus the intensity of vibration provided to the user can be increased.

According to the embodiments of the present invention, in a vibration module, a radius of curvature of a damper of an elastic member that performs a vibration function can be partially changed, and thus damage to the vibration module according to a period of use can be prevented.

According to the embodiments of the present invention, it is possible to effectively absorb skin care products into the user's skin or remove waste from the user's skin by performing a cooling and heating function together with adjusting vibration frequency control.

Effects of the present invention are not limited to the above-described effects and other unmentioned effects may be clearly understood by those skilled in the art from this specification and the following descriptions.

In the above, the configuration and features of the present invention have been described based on the embodiments of the present invention, but the present invention is not limited thereto.

## Claims

1. A vibration apparatus using acoustic wave, the vibration apparatus comprising:
a vibration module (1000) comprising a vibration generating unit (1100) and a vibration amplification unit (1200); and
wherein the vibration generating unit (1100) includes a housing (1170) providing an inner space, a magnet (1110) located in the housing (1170), a bobbin (1120) located apart from the magnet (1110) in a direction of axis (CA) of the magnet (1110), a coil (1130) disposed at an outer peripheral surface of the bobbin (1120) and an elastic member (1140) located on one side of the bobbin (1120),
wherein the vibration amplification unit (1200) includes a probe (1210) connected to the bobbin (1120) and vibrating in the direction of a central axis (CA) of the bobbin (1120),
a vibration transmission module (200) configured to couple to the housing (1170) directly or indirectly and surround at least a part of the probe (1210),
wherein the probe (1210) has a predetermined weight to amplify the vibration generated by the vibration generating unit (1100), **characterized in that:**
the vibration transmission module (200) includes a first vibration transmission module (200a) formed to surround at least part of the probe(1210) and a second vibration transmission module (200b) formed to surround at least part of the housing (1170),
the second vibration transmission module (200b) includes a plurality of coupling members (210b) capable of being coupled to at least part of the housing (1170),
the first vibration transmission module (200a) is connected to the second vibration transmission module (200b),
the first vibration transmission module (200a) is formed apart from the vibration generating unit (1100),
the vibration generated by the vibration generating unit (1100) is transmitted to the first vibration transmission module (200a) through the second vibration transmission module (200b),
at least part of an upper part of the vibration transmission module (200) is opened, and
wherein the height of the vibration transmission module (200) is set as a distance (a) from a virtual plane (P) perpendicular to a central axis (CA) of the bobbin (1120) to an end of the probe (1210) when the position of the bobbin (1120) is raised to the maximum by the vibration of the bobbin (1120) is shorter than a distance (b3) from the virtual plane (P) to an end of the upper part of the vibration transmission module (200) to transmit vibration indirectly by the probe (1210).

2. The vibration apparatus according to claim 1,
wherein a height of the vibration transmission module (200) is set as a distance from a virtual plane (P) perpendicular to a central axis (CA) of the bobbin (1120) to an end of the probe (1210) when the position of the bobbin (1120) is lowered to the maximum by the vibration of the bobbin (1120) is shorter than a distance from the virtual plane (P) to an end of the upper part of the vibration transmission module (200) to induce dispersion of the external force applied to the probe (1210) to the vibration transmission module (200).

3. The vibration apparatus according to claim 1,
wherein the vibration transmission module (200) is coupled to the housing (1170) such that the vibration generated by the vibration generating unit (1100) is transmitted to the vibration transmission module (200) through the housing (1170).

4. The vibration apparatus according to claim 1,
wherein the vibration apparatus comprises a vibration receiving unit (S) configured to surround at least part of the vibration transmission module (200),
wherein, the vibration transmission module (200) is contacted to the vibration receiving unit (S) such that the vibration amplified by the vibration amplification unit (1200) transmits to the vibration receiving unit (S), and
wherein, the vibration receiving unit (S) is not directly connected to the vibration amplification unit (1200).

5. The vibration apparatus according to claim 4,
wherein, the vibration receiving unit (S) includes at least one of the vibration modules (1000), and
wherein, the probe (1210) is placed in the vibration receiving unit (S) so that the direction of vibration of the probe (1210) corresponds to the height direction of the vibration receiving unit (S).

6. The vibration apparatus according to claim 1,
wherein the vibration apparatus comprises a vibration receiving unit (S) configured to surround at least part of the vibration transmission module (200),
wherein, the vibration transmission module (200) is contacted to the vibration receiving unit (S) such that the vibration amplified by the vibration amplification unit (1200) transmits to the vibration receiving unit (S),
wherein, the vibration receiving unit (S) is not directly connected to the vibration amplification unit (1200), and
wherein, the vibration transmission module (200) is located inside the vibration receiving unit (S).

7. The vibration apparatus according to claim 6,
wherein the vibration apparatus comprises a connecting shaft that penetrates the elastic member (1140), the bobbin (1120), the magnet (1110) and the housing (1170), and
wherein, the connecting shaft further comprises the probe (1210) vibrating in a vertical direction corresponding to the bobbin (1120) at the lower part of the connecting shaft.

8. The vibration apparatus according to claim 7,
wherein the probe (1210) is formed so that a plurality of wires of an elastic material are radially spread.

## Patentansprüche

1. Schwingungsvorrichtung, die Schallwellen verwendet, wobei die Schwingungsvorrichtung aufweist:
ein Schwingungsmodul (1000), das eine Schwingungserzeugungseinheit (1100) und eine Schwingungsverstärkungseinheit (1200) aufweist; und
wobei die Schwingungserzeugungseinheit (1100) ein Gehäuse (1170), das einen Innenraum bereitstellt, einen Magneten (1110), der in dem Gehäuse (1170) angeordnet ist, einen Spulenkörper (1120), der in einer Richtung der Achse (CA) des Magneten (1110) von dem Magneten (1110) entfernt angeordnet ist, eine Spule (1130), die an einer äußeren Umfangsfläche des Spulenkörpers (1120) angeordnet ist, und ein elastisches Element (1140) umfasst, das auf einer Seite des Spulenkörpers (1120) angeordnet ist,
wobei die Schwingungsverstärkungseinheit (1200) eine Sonde (1210) umfasst, die mit dem Spulenkörper (1120) verbunden ist und in Richtung einer Mittelachse (CA) des Spulenkörpers (1120) schwingt,
ein Schwingungsübertragungsmodul (200), das so konfiguriert ist, dass es direkt oder indirekt mit dem Gehäuse (1170) gekoppelt ist und zumindest einen Teil der Sonde (1210) umgibt,
wobei die Sonde (1210) ein vorbestimmtes Gewicht hat, um die von der Schwingungserzeugungseinheit (1100) erzeugte Schwingung zu verstärken, **dadurch gekennzeichnet, dass**
das Schwingungsübertragungsmodul (200) ein erstes Schwingungsübertagungsmodul (200a), das so ausgebildet ist, dass es zumindest einen Teil der Sonde (1210) umgibt, und ein zweites Schwingungsübertragungsmodul (200b) umfasst, das so ausgebildet ist, dass es zumindest einen Teil des Gehäuses (1170) umgibt,
das zweite Schwingungsübertragungsmodul (200b) mehrere Kopplungselemente (210b) umfasst, die mit mindestens einem Teil des Gehäuses (1170) gekoppelt werden können, das erste Schwingungsübertragungsmodul (200a) mit dem zweiten Schwingungsübertragungsmodul (200b) verbunden ist,
das erste Schwingungsübertragungsmodul (200a) getrennt von der Schwingungserzeugungseinheit (1100) ausgebildet ist,
die von der Schwingungserzeugungseinheit (1100) erzeugte Schwingung durch das zweite Schwingungsübertragungsmodul (200b) auf das erste Schwingungsübertragungsmodul (200a) übertragen wird,
zumindest ein Teil eines oberen Teils des Schwingungsübertragungsmoduls (200) geöffnet ist, und
wobei die Höhe des Schwingungsübertragungsmoduls (200), die als ein Abstand (a) von einer virtuellen Ebene (P) senkrecht zu einer Mittelachse (CA) des Spulenkörpers (1120) zu einem Ende der Sonde (1210) festgelegt ist, wenn die Position des Spulenköpers (1120) durch die Schwingung des Spulenkörpers (1120) auf das Maximum angehoben ist, kürzer ist als ein Abstand (b3) von der virtuellen Ebene (P) zu einem Ende des oberen Teils des Schwingungsübertragungsmoduls (200), um Schwingung indirekt durch die Sonde (1210) zu übertragen.

2. Schwingungsvorrichtung nach Anspruch 1,
wobei eine Höhe des Schwingungsübertragungsmoduls (200), die als ein Abstand von einer virtuellen Ebene (P) senkrecht zu einer Mittelachse (CA) des Spulenkörpers (1120) zu einem Ende der Sonde (1210) festgelegt ist, wenn die Position des Spulenköpers (1120) durch die Schwingung des Spulenkörpers (1120) auf das Maximum abgesenkt ist, kürzer ist als ein Abstand von der virtuellen Ebene (P) zu einem Ende des oberen Teils des Schwingungsübertragungsmoduls (200), um die Verteilung der auf die Sonde (1210) ausgeübten äußeren Kraft auf das Schwingungsübertragungsmodul (200) zu induzieren.

3. Schwingungsvorrichtung nach Anspruch 1,
wobei das Schwingungsübertragungsmodul (200) mit dem Gehäuse (1170) gekoppelt ist, so dass die von der Schwingungserzeugungseinheit (1100) erzeugte Schwingung durch das Gehäuse (1170) auf das Schwingungsübertragungsmodul (200) übertragen wird.

4. Schwingungsvorrichtung nach Anspruch 1,
wobei die Schwingungsvorrichtung eine Schwingungsempfangseinheit (S) aufweist, die so konfiguriert ist, dass sie zumindest einen Teil des Schwingungsübertragungsmoduls (200) umgibt,
wobei das Schwingungsübertragungsmodul (200) mit der Schwingungsempfangseinheit (S) in Kontakt steht, so dass die von der Schwingungsverstärkungseinheit (1200) verstärkte Schwingung an die Schwingungsempfangseinheit (S) übertragen wird, und
wobei die Schwingungsempfangseinheit (S) nicht direkt mit der Schwingungsverstärkungseinheit (1200) verbunden ist.

5. Schwingungsvorrichtung nach Anspruch 4,
wobei die Schwingungsempfangseinheit (S) mindestens eines der Schwingungsmodule (1000) umfasst, und
wobei die Sonde (1210) so in der Schwingungsempfangseinheit (S) angeordnet ist, dass die Schwingungsrichtung der Sonde (1210) der Höhenrichtung der Schwingungsempfangseinheit (S) entspricht.

6. Schwingungsvorrichtung nach Anspruch 1,
wobei die Schwingungsvorrichtung eine Schwingungsempfangseinheit (S) aufweist, die so konfiguriert ist, dass sie zumindest einen Teil des Schwingungsübertragungsmoduls (200) umgibt,
wobei das Schwingungsübertragungsmodul (200) mit der Schwingungsempfangseinheit (S) in Kontakt steht, so dass die von der Schwingungsverstärkungseinheit (1200) verstärkte Schwingung an die Schwingungsempfangseinheit (S) übertragen wird, wobei die Schwingungsempfangseinheit (S) nicht direkt mit der Schwingungsverstärkungseinheit (1200) verbunden ist, und
wobei sich das Schwingungsübertagungsmodul (200) innerhalb der Schwingungsempfangseinheit (S) befindet.

7. Schwingungsvorrichtung nach Anspruch 6,
wobei die Schwingungsvorrichtung eine Verbindungswelle aufweist, die das elastische Element (1140), den Spulenkörper (1120), den Magneten (1110) und das Gehäuse (1170) durchdringt, und
wobei die Verbindungswelle ferner die Sonde (1210) aufweist, die in einer vertikalen Richtung, entsprechend dem Spulenkörper (1120), am unteren Teil der Verbindungswelle schwingt.

8. Schwingungsvorrichtung nach Anspruch 7,
wobei die Sonde (1210) so geformt ist, dass mehrere Drähte aus einem elastischen Material radial verteilt sind.

## Revendications

1. Appareil de vibration utilisant une onde acoustique, l'appareil de vibration comprenant :
un module de vibration (1000) comprenant une unité de génération de vibration (1100) et une unité d'amplification de vibration (1200) ; et
dans lequel l'unité de génération de vibration (1100) comporte un boîtier (1170) fournissant un espace intérieur, un aimant (1110) situé dans le boîtier (1170), une bobine (1120) située à l'écart de l'aimant (1110) dans une direction d'axe (CA) de l'aimant (1110), un enroulement (1130) disposé au niveau d'une surface périphérique extérieur de la bobine (1120) et un élément élastique (1140) situé sur un côté de la bobine (1120),
dans lequel l'unité d'amplification de vibration (1200) comporte une sonde (1210) connectée à la bobine (1120) et vibrant dans la direction d'un axe central (CA) de la bobine (1120),
un module de transmission de vibration (200) configuré pour se coupler au boîtier (1170) directement ou indirectement et entourer au moins une partie de la sonde (1210),
dans lequel la sonde (1210) a un poids prédéterminé pour amplifier la vibration générée par l'unité de génération de vibration (1100), **caractérisé en ce que** :
le module de transmission de vibration (200) comporte un premier module de transmission de vibration (200a) formé pour entourer au moins une partie de la sonde (1210) et un deuxième module de transmission de vibration (200b) formé pour entourer au moins une partie du boîtier (1170),
le deuxième module de transmission de vibration (200b) comporte une pluralité d'éléments de couplage (210b) capables d'être couplés à au moins une partie du boîtier (1170),
le premier module de transmission de vibration (200a) est connecté au deuxième module de transmission de vibration (200b),
le premier module de transmission de vibration (200a) est formé à l'écart de l'unité de génération de vibration (1100),
la vibration générée par l'unité de génération de vibration (1100) est transmise au premier module de transmission de vibration (200a) via le deuxième module de transmission de vibration (200b),
au moins une partie d'une partie supérieure du module de transmission de vibration (200) est ouverte, et
dans lequel la hauteur du module de transmission de vibration (200) est définie comme une distance (a) allant d'un plan virtuel (P) perpendiculaire à un axe central (CA) de la bobine (1120) jusqu'à une extrémité de la sonde (1210) lorsque la position de la bobine (1120) est élevée au maximum par la vibration de la bobine (1120), qui est inférieure à une distance (b3) allant du plan virtuel (P) jusqu'à une extrémité de la partie supérieure du module de transmission de vibration (200) pour transmettre indirectement une vibration par la sonde (1210).

2. Appareil de vibration selon la revendication 1,
dans lequel une hauteur du module de transmission de vibration (200) est définie comme une distance allant d'un plan virtuel (P) perpendiculaire à un axe central (CA) de la bobine (1120) jusqu'à une extrémité de la sonde (1210) lorsque la position de la bobine (1120) est abaissée au maximum par la vibration de la bobine (1120), qui est inférieure à une distance allant du plan virtuel (P) jusqu'à une extrémité de la partie supérieure du module de transmission de vibration (200) pour induire une dispersion de la force externe appliquée à la sonde (1210) vers le module de transmission de vibration (200).

3. Appareil de vibration selon la revendication 1,
dans lequel le module de transmission de vibration (200) est couplé au boîtier (1170) de telle sorte que la vibration générée par l'unité de génération de vibration (1100) est transmise au module de transmission de vibration (200) à travers le boîtier (1170).

4. Appareil de vibration selon la revendication 1,
dans lequel l'appareil de vibration comprend une unité de réception de vibration (S) configurée pour entourer au moins une partie du module de transmission de vibration (200),
dans lequel le module de transmission de vibration (200) est mis en contact avec l'unité de réception de vibration (S) de telle sorte que la vibration amplifiée par l'unité d'amplification de vibration (1200) est transmise à l'unité de réception de vibration (S), et
dans lequel l'unité de réception de vibration (S) n'est pas directement connectée à l'unité d'amplification de vibration (1200).

5. Appareil de vibration selon la revendication 4,
dans lequel l'unité de réception de vibration (S) comporte au moins l'un des modules de vibration (1000), et
dans lequel la sonde (1210) est placée dans l'unité de réception de vibration (S) de sorte que la direction de vibration de la sonde (1210) correspond à la direction de hauteur de l'unité de réception de vibration (S).

6. Appareil de vibration selon la revendication 1,
dans lequel l'appareil de vibration comprend une unité de réception de vibration (S) configurée pour entourer au moins une partie du module de transmission de vibration (200),
dans lequel le module de transmission de vibration (200) est mis en contact avec l'unité de réception de vibration (S) de telle sorte que la vibration amplifiée par l'unité d'amplification de vibration (1200) est transmise à l'unité de réception de vibration (S),
dans lequel l'unité de réception de vibration (S) n'est pas directement connectée à l'unité d'amplification de vibration (1200), et
dans lequel le module de transmission de vibration (200) est situé à l'intérieur de l'unité de réception de vibration (S).

7. Appareil de vibration selon la revendication 6,
dans lequel l'appareil de vibration comprend un arbre de connexion qui pénètre dans l'élément élastique (1140), la bobine (1120), l'aimant (1110) et le boîtier (1170), et
dans lequel l'arbre de connexion comporte en outre la sonde (1210) vibrant dans une direction verticale correspondant à la bobine (1120) au niveau de la partie inférieure de l'arbre de connexion.

8. Appareil de vibration selon la revendication 7,
dans lequel la sonde (1210) est formée de telle sorte qu'une pluralité de fils d'un matériau élastique sont répartis radialement.
